# EUROPEAN PATENT APPLICATION

(11) **EP 4 772 234 A2**
(43) Date of publication of application: **08.07.2026**
(21) Application number: 26165271.3
(22) Date of filing: 17.07.2023
(51) Int. Cl.: A61N 1/365

(54) **ACUTE NON-EXCITATORY ELECTRICAL HEART FAILURE THERAPY**

(30) Priority: 18.07.2022 US 202263389967 P
(62) Divisional of application: 23749160.0
(71) Applicant: Impulse Dynamics NV, Willemstad (CW)
(72) Inventor: PRUTCHI, David, Voorhees, New Jersey, 08043 (US); BEN DAVID, Tamir, 6998827 Tel-Aviv (IL)
(74) Representative: Cohausz & Florack

(57) **Abstract**

An aspect of some embodiments of the invention relates to providing acute non-excitatory electrical heart failure therapy to a patient according to one or more criteria. Exemplary criteria are one or more of suffering from an acute angina episode, a patient after a myocardial infraction episode, a patient after heart surgery, a patient that is already on a Cath-Lab and would be beneficial to see if the treatment could help the patient, patients that suffer from heart failure and optionally already receive chronic non-excitatory electrical heart failure therapy, a patient that requires an improvement in blood flow, patients that show positive results from trial activation of acute non-excitatory electrical heart failure therapy, patients that require assistance in performing daily activities, like exercise, social events, after taking certain drugs, performing intercourse, sleeping and suffer from emotional stress.

## Description

### RELATED APPLICATION/S

This application claims the benefit of priority of U.S. Provisional Patent Application No. 63/389,967 filed on 18 July 2022, the contents of which are incorporated herein by reference in their entirety.

### FIELD AND BACKGROUND OF THE INVENTION

The present invention, in some embodiments thereof, relates to non-excitatory electrical heart failure therapy and, more particularly, but not exclusively, to acute non-excitatory electrical heart failure therapy.

Additional background art includes U.S. Patent No. US8311629B2 disclosing a method of modifying the force of contraction of at least a portion of a heart chamber, including providing a subject having a heart, comprising at least a portion having an activation, and applying a non-excitatory electric field having a given duration, at a delay after the activation, to the portion, which causes the force of contraction to be increased by a least 5%.

### SUMMARY OF THE INVENTION

Following is a non-exclusive list including some examples of embodiments of the invention. The invention also includes embodiments which include fewer than all the features in an example and embodiments using features from multiple examples, also if not expressly listed below.

Example 1. A system for cardiac stimulation, comprising an implantable controller, said controller configured to carry out the method of:
a. receiving an indication that a patient is suffering of an acute angina event;
b. providing an effective amount of non-excitatory electrical heart failure therapy.

Example 2. The system according to example 1, wherein said system further comprises at least one sensor configured for detecting said acute angina event; and wherein said indication is received from said at least one sensor.

Example 3. The system according to example 2, wherein said at least one sensor is located within said system and implanted within a body of said patient.

Example 4. The system according to example 2, wherein said at least one sensor is located outside a body of said patient and wherein said indication is transmitted to said implantable controller.

Example 5. The system according to any one of examples 1-4, wherein said indication is provided to said implantable controller by an external source.

Example 6. The system according to example 5, wherein said external source is one or more of a physician, said patient and an external device.

Example 7. The system according to any one of examples 1-6, wherein said effective amount of non-excitatory electrical heart failure therapy comprises providing a pulse amplitude of 7.5V, a pulse width 5mS and two bipolar pulses per cardiac cycle.

Example 8. The system according to any one of examples 1-7, wherein said providing comprises providing said effective amount of non-excitatory electrical heart failure therapy for at least 5 hours of stimulation per day.

Example 9. The system according to any one of examples 1-8, wherein said providing comprises increasing a total of number of pulses provided per day an increment from about 5% to about 200%.

Example 10. The system according to any one of examples 1-9, wherein said providing comprises increasing a total of number of pulses provided per hour an increment from about 5% to about 200%.

Example 11. The system according to any one of examples 1-10, wherein said providing comprises increasing a pulse amplitude of pulses provided an increment from about 5% to about 200%.

Example 12. The system according to any one of examples 1-11, wherein said providing comprises increasing a pulse width of pulses provided an increment from about 5% to about 200%.

Example 13. The system according to any one of examples 1-12, wherein said providing comprises increasing a number of pulses provided per cardiac cycle an increment from about 5% to about 200%.

Example 14. The system according to any one of examples 9-13, wherein said increasing is performed automatically by an acute non-excitatory electrical heart failure device.

Example 15. The system according to any one of examples 9-13, wherein said increasing is performed manually by said patient.

Example 16. The system according to any one of examples 1-15, wherein said controller is further configured to receive an indication of an improvement in said acute angina event, and further configured for amending said effective amount of non-excitatory electrical heart failure therapy accordingly.

Example 17. A system for cardiac stimulation, comprising an implantable controller, said controller configured to carry out the method of:
a. receiving an indication that a patient had suffered a myocardial infraction event and/or had heart surgery;
b. providing an effective amount of non-excitatory electrical heart failure therapy.

Example 18. The system according to example 17, wherein said system further comprises at least one sensor configured for detecting said myocardial infraction event; and wherein said indication is received from said at least one sensor.

Example 19. The system according to example 18, wherein said at least one sensor is located within said system and implanted within a body of said patient.

Example 20. The system according to example 18, wherein said at least one sensor is located outside a body of said patient and wherein said indication is transmitted to said implantable controller.

Example 21. The system according to any one of examples 17-20, wherein said indication is provided to said implantable controller by an external source.

Example 22. The system according to example 21, wherein said external source is one or more of a physician, said patient and an external device.

Example 23. The system according to any one of examples 17-22, wherein said effective amount of non-excitatory electrical heart failure therapy comprises providing a pulse amplitude of 7.5V, a pulse width 5mS and two bipolar pulses per cardiac cycle.

Example 24. The system according to any one of examples 17-23, wherein said providing comprises providing said effective amount of non-excitatory electrical heart failure therapy for at least 5 hours of stimulation per day.

Example 25. The system according to any one of examples 17-24, wherein said providing comprises increasing a total of number of pulses provided per day an increment from about 5% to about 200%.

Example 26. The system according to any one of examples 17-25, wherein said providing comprises increasing a total of number of pulses provided per hour an increment from about 5% to about 200%.

Example 27. The system according to any one of examples 17-26, wherein said providing comprises increasing a pulse amplitude of pulses provided an increment from about 5% to about 200%.

Example 28. The system according to any one of examples 17-27, wherein said providing comprises increasing a pulse width of pulses provided an increment from about 5% to about 200%.

Example 29. The system according to any one of examples 17-28, wherein said providing comprises increasing a number of pulses provided per cardiac cycle an increment from about 5% to about 200%.

Example 30. The system according to any one of examples 25-29, wherein said increasing is performed automatically by an acute non-excitatory electrical heart failure device.

Example 31. The system according to any one of examples 25-29, wherein said increasing is performed manually by said patient.

Example 32. The system according to example 1, wherein said controller is further configured to receive an indication of an improvement in a status of said patient, and further configured for amending said effective amount of non-excitatory electrical heart failure therapy accordingly.

Example 33. A method of patient selection and/or treating a patient with acute non-excitatory electrical heart failure therapy, the method comprising:
a. identifying an eligible patient for acute non-excitatory electrical heart failure therapy;
b. providing means to perform acute non-excitatory electrical heart failure therapy;
c. performing test to validate positive responses to acute non-excitatory electrical heart failure therapy by providing acute non-excitatory electrical heart failure therapy to said patient;
d. assessing, based on a result of said test, that said patient positively responds to acute non-excitatory electrical heart failure therapy;
e. continuing providing an effective amount of non-excitatory electrical heart failure therapy.

Example 34. The method according to example 33, wherein said positive responses are positive physiological responses.

Example 35. The method according to example 34, wherein said positive physiological responses are one or more of: a change in stroke volume (SV), a change in Cardiac output (CO), a change of end-diastolic volume (EDV), a change in cardiac contractility that is measured by calculating the stroke volume divided by end-diastolic volume (SV/EDV), a change in cardiac tissue mechanical mobility and a flow rate in the coronary arteries.

Example 36. The method according to example 33, wherein said effective amount of non-excitatory electrical heart failure therapy comprises providing a pulse amplitude of 7.5V, a pulse width 5mS and two bipolar pulses per cardiac cycle.

Example 37. The method according to example 33, wherein said providing comprises providing said effective amount of non-excitatory electrical heart failure therapy for at least 5 hours of stimulation per day.

Example 38. The method according to example 33, wherein said providing comprises increasing a total of number of pulses provided per day an increment from about 5% to about 200%.

Example 39. The method according to example 33, wherein said providing comprises increasing a total of number of pulses provided per hour an increment from about 5% to about 200%.

Example 40. The method according to example 33, wherein said providing comprises increasing a pulse amplitude of pulses provided an increment from about 5% to about 200%.

Example 41. The method according to example 33, wherein said providing comprises increasing a pulse width of pulses provided an increment from about 5% to about 200%.

Example 42. The method according to example 33, wherein said providing comprises increasing a number of pulses provided per cardiac cycle an increment from about 5% to about 200%.

Example 43. The method according to any one of examples 38-42, wherein said increasing is performed automatically by an acute non-excitatory electrical heart failure device.

Example 44. The method according to any one of examples 38-42, wherein said increasing is performed manually by said patient.

Example 45. The method according to example 33, further comprising continuously evaluating positive responds in said patient, and further comprising amending said effective amount of non-excitatory electrical heart failure therapy accordingly.

Example 46. A system for cardiac stimulation, comprising an implantable controller, said controller configured to carry out the method of:
a. receiving an indication that a patient is in need of an increase in blood flow;
b. providing an effective amount of non-excitatory electrical heart failure therapy.

Example 47. The system according to example 46, wherein said system further comprises at least one sensor configured for detecting said need of an increase in blood flow; and wherein said indication is received from said at least one sensor.

Example 48. The system according to example 47, wherein said at least one sensor is located within said system and implanted within a body of said patient.

Example 49. The system according to example 47, wherein said at least one sensor is located outside a body of said patient and wherein said indication is transmitted to said implantable controller.

Example 50. The system according to any one of examples 46-49, wherein said indication is provided to said implantable controller by an external source.

Example 51. The system according to example 50, wherein said external source is one or more of a physician, said patient and an external device.

Example 52. The system according to any one of examples 46-51, wherein said effective amount of non-excitatory electrical heart failure therapy comprises providing a pulse amplitude of 7.5V, a pulse width 5mS and two bipolar pulses per cardiac cycle.

Example 53. The system according to any one of examples 46-52, wherein said providing comprises providing said effective amount of non-excitatory electrical heart failure therapy for at least 5 hours of stimulation per day.

Example 54. The system according to any one of examples 46-53, wherein said providing comprises increasing a total of number of pulses provided per day an increment from about 5% to about 200%.

Example 55. The system according to any one of examples 46-54, wherein said providing comprises increasing a total of number of pulses provided per hour an increment from about 5% to about 200%.

Example 56. The system according to any one of examples 46-55, wherein said providing comprises increasing a pulse amplitude of pulses provided an increment from about 5% to about 200%.

Example 57. The system according to any one of examples 46-56, wherein said providing comprises increasing a pulse width of pulses provided an increment from about 5% to about 200%.

Example 58. The system according to any one of examples 46-57, wherein said providing comprises increasing a number of pulses provided per cardiac cycle an increment from about 5% to about 200%.

Example 59. The system according to any one of examples 54-58, wherein said increasing is performed automatically by an acute non-excitatory electrical heart failure device.

Example 60. The system according to any one of examples 54-58, wherein said increasing is performed manually by said patient.

Example 61. The system according to any one of examples 46-60, wherein said controller is further configured to receive an indication of an improvement in said blood flow, and further configured for amending said effective amount of non-excitatory electrical heart failure therapy accordingly.

Example 62. The system according to any one of examples 46-61, wherein said controller is further configured to receive an indication of an erection status in said patient.

Example 63. A system for cardiac stimulation, comprising an implantable controller, said controller configured to carry out the method of:
a. receiving an indication that a patient has taken a specific pharmaceutical;
b. providing an effective amount of non-excitatory electrical heart failure therapy.

Example 64. The system according to example 63, wherein said system further comprises at least one sensor configured for detecting that said patient has taken said specific pharmaceutical; and wherein said indication is received from said at least one sensor.

Example 65. The system according to example 64, wherein said at least one sensor is located within said system and implanted within a body of said patient.

Example 66. The system according to example 64, wherein said at least one sensor is located outside a body of said patient and wherein said indication is transmitted to said implantable controller.

Example 67. The system according to any one of examples 63-66, wherein said indication is provided to said implantable controller by an external source.

Example 68. The system according to example 67, wherein said external source is one or more of a physician, said patient and an external device.

Example 69. The system according to any one of examples 63-68, wherein said effective amount of non-excitatory electrical heart failure therapy comprises providing a pulse amplitude of 7.5V, a pulse width 5mS and two bipolar pulses per cardiac cycle.

Example 70. The system according to any one of examples 63-69, wherein said providing comprises providing said effective amount of non-excitatory electrical heart failure therapy for at least 5 hours of stimulation per day.

Example 71. The system according to any one of examples 63-70, wherein said providing comprises increasing a total of number of pulses provided per day an increment from about 5% to about 200%.

Example 72. The system according to any one of examples 63-71, wherein said providing comprises increasing a total of number of pulses provided per hour an increment from about 5% to about 200%.

Example 73. The system according any one of examples 63-72, wherein said providing comprises increasing a pulse amplitude of pulses provided an increment from about 5% to about 200%.

Example 74. The system according to any one of examples 63-73, wherein said providing comprises increasing a pulse width of pulses provided an increment from about 5% to about 200%.

Example 75. The system according to any one of examples 63-74, wherein said providing comprises increasing a number of pulses provided per cardiac cycle an increment from about 5% to about 200%.

Example 76. The system according to any one of examples 71-75, wherein said increasing is performed automatically by an acute non-excitatory electrical heart failure device.

Example 77. The system according to any one of examples 71-75, wherein said increasing is performed manually by said patient.

Example 78. The system according to any one of examples 63-77, wherein said controller is further configured to receive an indication of an improvement in a status in said patient, and further configured for amending said effective amount of non-excitatory electrical heart failure therapy accordingly.

Example 79. The system according to any one of examples 63-78, wherein said controller is further configured to receive an indication of an erection status in said patient.

Example 80. A system for cardiac stimulation, comprising an implantable controller, said controller configured to carry out the method of:
a. receiving an indication that a patient is suffering from an increase in stress levels;
b. providing an effective amount of non-excitatory electrical heart failure therapy.

Example 81. The system according to example 80, wherein said system further comprises at least one sensor configured for detecting said increase in stress levels; and wherein said indication is received from said at least one sensor.

Example 82. The system according to example 81, wherein said at least one sensor is located within said system and implanted within a body of said patient.

Example 83. The system according to example 81, wherein said at least one sensor is located outside a body of said patient and wherein said indication is transmitted to said implantable controller.

Example 84. The system according to any one of examples 80-83, wherein said indication is provided to said implantable controller by an external source.

Example 85. The system according to example 84, wherein said external source is one or more of a physician, said patient and an external device.

Example 86. The system according to any one of examples 80-85, wherein said effective amount of non-excitatory electrical heart failure therapy comprises providing a pulse amplitude of 7.5V, a pulse width 5mS and two bipolar pulses per cardiac cycle.

Example 87. The system according to any one of examples 80-86, wherein said providing comprises providing said effective amount of non-excitatory electrical heart failure therapy for at least 5 hours of stimulation per day.

Example 88. The system according to any one of examples 80-87, wherein said providing comprises increasing a total of number of pulses provided per day an increment from about 5% to about 200%.

Example 89. The system according to any one of examples 80-88, wherein said providing comprises increasing a total of number of pulses provided per hour an increment from about 5% to about 200%.

Example 90. The system according to any one of examples 80-89, wherein said providing comprises increasing a pulse amplitude of pulses provided an increment from about 5% to about 200%.

Example 91. The system according to any one of examples 80-90, wherein said providing comprises increasing a pulse width of pulses provided an increment from about 5% to about 200%.

Example 92. The system according to any one of examples 80-91, wherein said providing comprises increasing a number of pulses provided per cardiac cycle an increment from about 5% to about 200%.

Example 93. The system according to any one of examples 80-92, wherein said increasing is performed automatically by an acute non-excitatory electrical heart failure device.

Example 94. The system according to any one of examples 80-93, wherein said increasing is performed manually by said patient.

Example 95. The system according to any one of examples 80-94, wherein said controller is further configured to receive an indication of an improvement in said stress levels, and further configured for amending said effective amount of non-excitatory electrical heart failure therapy accordingly.

Example 96. The system according to any one of examples 80-95, wherein said controller is further configured to receive an indication of an erection status in said patient.

Example 97. A system for cardiac stimulation, comprising an implantable controller, said controller configured to carry out the method of:
a. receiving an indication that a patient is suffering of a change in temperature within a predetermined range;
b. providing an effective amount of non-excitatory electrical heart failure therapy.

Example 98. The system according to example 97, wherein said system further comprises at least one sensor configured for detecting said change in temperature; and wherein said indication is received from said at least one sensor.

Example 99. The system according to example 98, wherein said at least one sensor is located within said system and implanted within a body of said patient.

Example 100. The system according to example 98, wherein said at least one sensor is located outside a body of said patient and wherein said indication is transmitted to said implantable controller.

Example 101. The system according to any one of examples 97-100, wherein said indication is provided to said implantable controller by an external source.

Example 102. The system according to example 101, wherein said external source is one or more of a physician, said patient and an external device.

Example 103. The system according to any one of examples 97-102, wherein said effective amount of non-excitatory electrical heart failure therapy comprises providing a pulse amplitude of 7.5V, a pulse width 5mS and two bipolar pulses per cardiac cycle.

Example 104. The system according to any one of examples 97-103, wherein said providing comprises providing said effective amount of non-excitatory electrical heart failure therapy for at least 5 hours of stimulation per day.

Example 105. The system according to any one of examples 97-104, wherein said providing comprises increasing a total of number of pulses provided per day an increment from about 5% to about 200%.

Example 106. The system according to any one of examples 97-105, wherein said providing comprises increasing a total of number of pulses provided per hour an increment from about 5% to about 200%.

Example 107. The system according to any one of examples 97-106, wherein said providing comprises increasing a pulse amplitude of pulses provided an increment from about 5% to about 200%.

Example 108. The system according to any one of examples 97-107, wherein said providing comprises increasing a pulse width of pulses provided an increment from about 5% to about 200%.

Example 109. The system according to any one of examples 97-108, wherein said providing comprises increasing a number of pulses provided per cardiac cycle an increment from about 5% to about 200%.

Example 110. The system according to any one of examples 105-109, wherein said increasing is performed automatically by an acute non-excitatory electrical heart failure device.

Example 111. The system according to any one of examples 105-110, wherein said increasing is performed manually by said patient.

Example 112. The system according to any one of examples 97-111, wherein said controller is further configured to receive an indication of an improvement in said temperature, and further configured for amending said effective amount of non-excitatory electrical heart failure therapy accordingly.

Example 113. A system for cardiac stimulation, comprising an implantable controller, said controller configured to carry out the method of:
a. receiving an indication that a patient is suffering from an acute response event to a previously provided amount of acute non-excitatory electrical heart failure therapy;
b. amending at least one parameter of said previously provided amount of acute non-excitatory electrical heart failure therapy;
c. providing an effective amount of amended non-excitatory electrical heart failure therapy.

Example 114. The system according to example 113, wherein said system further comprises at least one sensor configured for detecting said acute response event; and wherein said indication is received from said at least one sensor.

Example 115. The system according to example 114, wherein said at least one sensor is located within said system and implanted within a body of said patient.

Example 116. The system according to example 114, wherein said at least one sensor is located outside a body of said patient and wherein said indication is transmitted to said implantable controller.

Example 117. The system according to any one of examples 113-116, wherein said indication is provided to said implantable controller by an external source.

Example 118. The system according to example 117, wherein said external source is one or more of a physician, said patient and an external device.

Example 119. The system according to any one of examples 113-118, wherein said effective amount of amended non-excitatory electrical heart failure therapy comprises providing a pulse amplitude of 7.5V, a pulse width 5mS and two bipolar pulses per cardiac cycle.

Example 120. The system according to any one of examples 113-119, wherein said providing comprises providing said effective amount of amended non-excitatory electrical heart failure therapy for at least 5 hours of stimulation per day.

Example 121. The system according to any one of examples 113-120, wherein said providing comprises increasing a total of number of pulses provided per day an increment from about 5% to about 200%.

Example 122. The system according to any one of examples 113-121, wherein said providing comprises increasing a total of number of pulses provided per hour an increment from about 5% to about 200%.

Example 123. The system according to any one of examples 113-122, wherein said providing comprises increasing a pulse amplitude of pulses provided an increment from about 5% to about 200%.

Example 124. The system according to any one of examples 113-123, wherein said providing comprises increasing a pulse width of pulses provided an increment from about 5% to about 200%.

Example 125. The system according to any one of examples 113-124, wherein said providing comprises increasing a number of pulses provided per cardiac cycle an increment from about 5% to about 200%.

Example 126. The system according to any one of examples 121-125, wherein said increasing is performed automatically by an acute non-excitatory electrical heart failure device.

Example 127. The system according to any one of examples 121-126, wherein said increasing is performed manually by said patient.

Example 128. The system according to any one of examples 113-127, wherein said controller is further configured to receive an indication of an improvement in said acute response event, and configured for amending again said effective amount of non-excitatory electrical heart failure therapy accordingly.

Example 1a. A method of treating a patient with acute non-excitatory electrical heart failure therapy, the method comprising:
a. identifying a patient that suffers of acute angina event;
b. providing an effective amount of non-excitatory electrical heart failure therapy.

Example 2a. The method according to example 1a, wherein said effective amount of non-excitatory electrical heart failure therapy comprises providing a pulse amplitude of 7.5V, a pulse width 5mS and two bipolar pulses per cardiac cycle.

Example 3a. The method according to example 1a, wherein said providing comprises providing said effective amount of non-excitatory electrical heart failure therapy for at least 5 hours of stimulation per day.

Example 4a. The method according to example 1a, wherein said providing comprises increasing a total of number of pulses provided per day an increment from about 5% to about 200%.

Example 5a. The method according to example 1a, wherein said providing comprises increasing a total of number of pulses provided per hour an increment from about 5% to about 200%.

Example 6a. The method according to example 1a, wherein said providing comprises increasing a pulse amplitude of pulses provided an increment from about 5% to about 200%.

Example 7a. The method according to example 1a, wherein said providing comprises increasing a pulse width of pulses provided an increment from about 5% to about 200%.

Example 8a. The method according to example 1a, wherein said providing comprises increasing a number of pulses provided per cardiac cycle an increment from about 5% to about 200%.

Example 9a. The method according to any one of examples 4a-8a, wherein said increasing is performed automatically by an acute non-excitatory electrical heart failure device.

Example 10a. The method according to any one of examples 4a-8a, wherein said increasing is performed manually by said patient.

Example 11a. The method according to example 1, further comprising evaluating an improvement in said acute angina event, and further comprising amending said effective amount of non-excitatory electrical heart failure therapy accordingly.

Example 12a. A method of treating a patient with acute non-excitatory electrical heart failure therapy, the method comprising:
a. identifying a patient that had suffered a myocardial infraction event and/or had heart surgery;
b. providing an effective amount of non-excitatory electrical heart failure therapy.

Example 13a. The method according to example 12a, wherein said effective amount of non-excitatory electrical heart failure therapy comprises providing a pulse amplitude of 7.5V, a pulse width 5mS and two bipolar pulses per cardiac cycle.

Example 14a. The method according to example 12a, wherein said providing comprises providing said effective amount of non-excitatory electrical heart failure therapy for at least 5 hours of stimulation per day.

Example 15a. The method according to example 12a, wherein said providing comprises increasing a total of number of pulses provided per day an increment from about 5% to about 200%.

Example 16a. The method according to example 12a, wherein said providing comprises increasing a total of number of pulses provided per hour an increment from about 5% to about 200%.

Example 17a. The method according to example 12a, wherein said providing comprises increasing a pulse amplitude of pulses provided an increment from about 5% to about 200%.

Example 18a. The method according to example 12a, wherein said providing comprises increasing a pulse width of pulses provided an increment from about 5% to about 200%.

Example 19a. The method according to example 12a, wherein said providing comprises increasing a number of pulses provided per cardiac cycle an increment from about 5% to about 200%.

Example 20a. The method according to any one of examples 15a-19a, wherein said increasing is performed automatically by an acute non-excitatory electrical heart failure device.

Example 21a. The method according to any one of examples 15a-19a, wherein said increasing is performed manually by said patient.

Example 22a. The method according to example 12a, further comprising evaluating an improvement in a status of said patient, and further comprising amending said effective amount of non-excitatory electrical heart failure therapy accordingly.

Example 23a. A method of treating a patient with acute non-excitatory electrical heart failure therapy, the method comprising:
a. identifying an eligible patient for acute non-excitatory electrical heart failure therapy;
b. providing means to perform acute non-excitatory electrical heart failure therapy;
c. performing test to validate positive responses to acute non-excitatory electrical heart failure therapy by providing acute non-excitatory electrical heart failure therapy to said patient;
d. assessing, based on a result of said test, that said patient positively responds to acute non-excitatory electrical heart failure therapy;
e. continuing providing an effective amount of non-excitatory electrical heart failure therapy.

Example 24a. The method according to example 23a, wherein said positive responses are positive physiological responses.

Example 25a. The method according to example 24a, wherein said positive physiological responses are one or more of: a change in stroke volume (SV), a change in Cardiac output (CO), a change of end-diastolic volume (EDV), a change in cardiac contractility that is measured by calculating the stroke volume divided by end-diastolic volume (SV/EDV), a change in cardiac tissue mechanical mobility and a flow rate in the coronary arteries.

Example 26a. The method according to example 23a, wherein said effective amount of non-excitatory electrical heart failure therapy comprises providing a pulse amplitude of 7.5V, a pulse width 5mS and two bipolar pulses per cardiac cycle.

Example 27a. The method according to example 23a, wherein said providing comprises providing said effective amount of non-excitatory electrical heart failure therapy for at least 5 hours of stimulation per day.

Example 28a. The method according to example 23a, wherein said providing comprises increasing a total of number of pulses provided per day an increment from about 5% to about 200%.

Example 29a. The method according to example 23a, wherein said providing comprises increasing a total of number of pulses provided per hour an increment from about 5% to about 200%.

Example 30a. The method according to example 23a, wherein said providing comprises increasing a pulse amplitude of pulses provided an increment from about 5% to about 200%.

Example 31a. The method according to example 23a, wherein said providing comprises increasing a pulse width of pulses provided an increment from about 5% to about 200%.

Example 32a. The method according to example 23a, wherein said providing comprises increasing a number of pulses provided per cardiac cycle an increment from about 5% to about 200%.

Example 33a. The method according to any one of examples 28a-32a, wherein said increasing is performed automatically by an acute non-excitatory electrical heart failure device.

Example 34a. The method according to any one of examples 28a-32a, wherein said increasing is performed manually by said patient.

Example 35a. The method according to example 23a, further comprising continuously evaluating positive responds in said patient, and further comprising amending said effective amount of non-excitatory electrical heart failure therapy accordingly.

Example 36a. A method of treating a patient with acute non-excitatory electrical heart failure therapy, the method comprising:
a. identifying a patient in need of an increase in blood flow;
b. providing an effective amount of non-excitatory electrical heart failure therapy.

Example 37a. The method according to example 36a, wherein said effective amount of non-excitatory electrical heart failure therapy comprises providing a pulse amplitude of 7.5V, a pulse width 5mS and two bipolar pulses per cardiac cycle.

Example 38a. The method according to example 36a, wherein said providing comprises providing said effective amount of non-excitatory electrical heart failure therapy for at least 5 hours of stimulation per day.

Example 39a. The method according to example 36a, wherein said providing comprises increasing a total of number of pulses provided per day an increment from about 5% to about 200%.

Example 40a. The method according to example 36a, wherein said providing comprises increasing a total of number of pulses provided per hour an increment from about 5% to about 200%.

Example 41a. The method according to example 36a, wherein said providing comprises increasing a pulse amplitude of pulses provided an increment from about 5% to about 200%.

Example 42a. The method according to example 36a, wherein said providing comprises increasing a pulse width of pulses provided an increment from about 5% to about 200%.

Example 43a. The method according to example 36a, wherein said providing comprises increasing a number of pulses provided per cardiac cycle an increment from about 5% to about 200%.

Example 44a. The method according to any one of examples 39a-43a, wherein said increasing is performed automatically by an acute non-excitatory electrical heart failure device.

Example 45a. The method according to any one of examples 39a-43a, wherein said increasing is performed manually by said patient.

Example 46a. The method according to example 36a, further comprising evaluating an improvement in said blood flow, and further comprising amending said effective amount of non-excitatory electrical heart failure therapy accordingly.

Example 47a. The method according to example 36a, further comprising evaluating an erection status in said patient.

Example 48a. A method of treating a patient with acute non-excitatory electrical heart failure therapy, the method comprising:
a. identifying that a patient has taken a specific pharmaceutical;
b. providing an effective amount of non-excitatory electrical heart failure therapy.

Example 49a. The method according to example 48a, wherein said effective amount of non-excitatory electrical heart failure therapy comprises providing a pulse amplitude of 7.5V, a pulse width 5mS and two bipolar pulses per cardiac cycle.

Example 50a. The method according to example 48a, wherein said providing comprises providing said effective amount of non-excitatory electrical heart failure therapy for at least 5 hours of stimulation per day.

Example 51a. The method according to example 48a, wherein said providing comprises increasing a total of number of pulses provided per day an increment from about 5% to about 200%.

Example 52a. The method according to example 48a, wherein said providing comprises increasing a total of number of pulses provided per hour an increment from about 5% to about 200%.

Example 53a. The method according to example 48a, wherein said providing comprises increasing a pulse amplitude of pulses provided an increment from about 5% to about 200%.

Example 54a. The method according to example 48a, wherein said providing comprises increasing a pulse width of pulses provided an increment from about 5% to about 200%.

Example 55a. The method according to example 48a, wherein said providing comprises increasing a number of pulses provided per cardiac cycle an increment from about 5% to about 200%.

Example 56a. The method according to any one of examples 51a-55a, wherein said increasing is performed automatically by an acute non-excitatory electrical heart failure device.

Example 57a. The method according to any one of examples 51a-55a, wherein said increasing is performed manually by said patient.

Example 58a. The method according to example 48a, further comprising evaluating an improvement in a status in said patient, and further comprising amending said effective amount of non-excitatory electrical heart failure therapy accordingly.

Example 59a. The method according to example 48a, further comprising evaluating an erection status in said patient.

Example 60a. A method of treating a patient with acute non-excitatory electrical heart failure therapy, the method comprising:
a. identifying an increase in stress levels in a patient;
b. providing an effective amount of non-excitatory electrical heart failure therapy.

Example 61a. The method according to example 60a, wherein said effective amount of non-excitatory electrical heart failure therapy comprises providing a pulse amplitude of 7.5V, a pulse width 5mS and two bipolar pulses per cardiac cycle.

Example 62a. The method according to example 60a, wherein said providing comprises providing said effective amount of non-excitatory electrical heart failure therapy for at least 5 hours of stimulation per day.

Example 63a. The method according to example 60a, wherein said providing comprises increasing a total of number of pulses provided per day an increment from about 5% to about 200%.

Example 64a. The method according to example 60a, wherein said providing comprises increasing a total of number of pulses provided per hour an increment from about 5% to about 200%.

Example 65a. The method according to example 60a, wherein said providing comprises increasing a pulse amplitude of pulses provided an increment from about 5% to about 200%.

Example 66a. The method according to example 60a, wherein said providing comprises increasing a pulse width of pulses provided an increment from about 5% to about 200%.

Example 67a. The method according to example 60a, wherein said providing comprises increasing a number of pulses provided per cardiac cycle an increment from about 5% to about 200%.

Example 68a. The method according to any one of examples 63a-67a, wherein said increasing is performed automatically by an acute non-excitatory electrical heart failure device.

Example 69a. The method according to any one of examples 63a-67a, wherein said increasing is performed manually by said patient.

Example 70a. The method according to example 60a, further comprising evaluating an improvement in said stress levels, and further comprising amending said effective amount of non-excitatory electrical heart failure therapy accordingly.

Example 71a. The method according to example 60a, further comprising evaluating an erection status in said patient.

Example 72a. A method of treating a patient with acute non-excitatory electrical heart failure therapy, the method comprising:
a. identifying a patient that suffers of a change in temperature within a predetermined range;
b. providing an effective amount of non-excitatory electrical heart failure therapy.

Example 73a. The method according to example 72a, wherein said effective amount of non-excitatory electrical heart failure therapy comprises providing a pulse amplitude of 7.5V, a pulse width 5mS and two bipolar pulses per cardiac cycle.

Example 74a. The method according to example 72a, wherein said providing comprises providing said effective amount of non-excitatory electrical heart failure therapy for at least 5 hours of stimulation per day.

Example 75a. The method according to example 72a, wherein said providing comprises increasing a total of number of pulses provided per day an increment from about 5% to about 200%.

Example 76a. The method according to example 72a, wherein said providing comprises increasing a total of number of pulses provided per hour an increment from about 5% to about 200%.

Example 77a. The method according to example 72a, wherein said providing comprises increasing a pulse amplitude of pulses provided an increment from about 5% to about 200%.

Example 78a. The method according to example 72a, wherein said providing comprises increasing a pulse width of pulses provided an increment from about 5% to about 200%.

Example 79a. The method according to example 72a, wherein said providing comprises increasing a number of pulses provided per cardiac cycle an increment from about 5% to about 200%.

Example 80a. The method according to any one of examples 75a-79a, wherein said increasing is performed automatically by an acute non-excitatory electrical heart failure device.

Example 81a. The method according to any one of examples 75a-79a, wherein said increasing is performed manually by said patient.

Example 82a. The method according to example 72a, further comprising evaluating an improvement in said temperature, and further comprising amending said effective amount of non-excitatory electrical heart failure therapy accordingly.

Example 83a. A method of treating a patient with acute non-excitatory electrical heart failure therapy, the method comprising:
a. identifying a patient that suffers of an acute response event to a previously provided amount of acute non-excitatory electrical heart failure therapy;
b. amending at least one parameter of said previously provided amount of acute non-excitatory electrical heart failure therapy;
c. providing an effective amount of amended non-excitatory electrical heart failure therapy.

Example 84a. The method according to example 83a, wherein said effective amount of amended non-excitatory electrical heart failure therapy comprises providing a pulse amplitude of 7.5V, a pulse width 5mS and two bipolar pulses per cardiac cycle.

Example 85a. The method according to example 83a, wherein said providing comprises providing said effective amount of amended non-excitatory electrical heart failure therapy for at least 5 hours of stimulation per day.

Example 86a. The method according to example 83a, wherein said providing comprises increasing a total of number of pulses provided per day an increment from about 5% to about 200%.

Example 87a. The method according to example 83a, wherein said providing comprises increasing a total of number of pulses provided per hour an increment from about 5% to about 200%.

Example 88a. The method according to example 83a, wherein said providing comprises increasing a pulse amplitude of pulses provided an increment from about 5% to about 200%.

Example 89a. The method according to example 83a, wherein said providing comprises increasing a pulse width of pulses provided an increment from about 5% to about 200%.

Example 90a. The method according to example 83a, wherein said providing comprises increasing a number of pulses provided per cardiac cycle an increment from about 5% to about 200%.

Example 91a. The method according to any one of examples 86a-90a, wherein said increasing is performed automatically by an acute non-excitatory electrical heart failure device.

Example 92a. The method according to any one of examples 86a-90a, wherein said increasing is performed manually by said patient.

Example 93. The method according to example 83, further comprising evaluating an improvement in said acute response event, and further comprising amending again said effective amount of non-excitatory electrical heart failure therapy accordingly.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Figure 1 is a flowchart of an exemplary method of treatment of a patient suffering an acute angina episode with acute non-excitatory electrical heart failure therapy, according to some embodiments of the invention;
Figure 2 is method of treatment of a patient with acute non-excitatory electrical heart failure therapy after suffering a myocardial infraction and/or heart surgery, according to some embodiments of the invention;
Figure 3 is a flowchart of an exemplary method of treatment of a patient with acute non-excitatory electrical heart failure therapy after said patient has shown positive physiological responses to acute non-excitatory electrical heart failure therapy, according to some embodiments of the invention;
Figure 4 is a flowchart of an exemplary method of treatment of a patient with acute non-excitatory electrical heart failure therapy to assist in performing daily actions, according to some embodiments of the invention;
Figure 5 is a flowchart of an exemplary method of treatment of a patient with acute non-excitatory electrical heart failure therapy after said patient has taken a determined pharmaceutical, according to some embodiments of the invention;
Figure 6 is a flowchart of an exemplary method of treatment of a patient with acute non-excitatory electrical heart failure therapy during sleep, according to some embodiments of the invention;
Figure 7 is a flowchart of an exemplary method of treatment of a patient with acute non-excitatory electrical heart failure therapy during emotional stress, according to some embodiments of the invention;
Figure 8a is a schematic block diagram of an exemplary cardiac therapy device, according to some embodiments of the invention;
Figure 8b is a schematic diagram showing components of an implantable cardiac device, according to some embodiments of the invention;
Figure 9a schematically illustrates an exemplary implanted device configured for applying cardiac electrical stimulation, according to some embodiments of the invention; and
Figure 9b schematically illustrates an exemplary cardiac device comprising a plurality of leads, according to some embodiments of the invention.

### DESCRIPTION OF SPECIFIC EMBODIMENTS OF THE INVENTION

The present invention, in some embodiments thereof, relates to relates to non-excitatory electrical heart failure therapy and, more particularly, but not exclusively, to acute non-excitatory electrical heart failure therapy.

### Overview

An aspect of some embodiments of the invention relates to providing acute non-excitatory electrical heart failure therapy to a patient according to one or more criteria. In some embodiments, the criteria are one or more of suffering from an acute angina episode, a patient after a myocardial infraction episode, a patient after heart surgery, a patient that is already on a Cath-Lab and would be beneficial to see if the treatment could help the patient, patients that suffer from heart failure and optionally already receive chronic non-excitatory electrical heart failure therapy, a patient that requires an improvement in blood flow, patients that show positive results from trial activation of acute non-excitatory electrical heart failure therapy, patients that require assistance in performing daily activities, like exercise, social events, after taking certain drugs, performing intercourse, sleeping and suffer from emotional stress; and patients that showed a change in their condition and providing acute non-excitatory electrical heart failure therapy would be beneficial. In some embodiments, the activation of the acute non-excitatory electrical heart failure therapy is characterized by five hours of stimulation per day, a pulse amplitude of 7.5V, a pulse width 5mS and two bipolar pulses per cardiac cycle. In some embodiments, activation of the acute non-excitatory electrical heart failure therapy is triggered by the patient. In some embodiments, activation of the acute non-excitatory electrical heart failure therapy is triggered automatically by the system according to predetermined rules.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary non-excitatory electrical heart failure therapy device

In some embodiments, a patient having or that suffered a heart failure event is implanted with a device that provides non-excitatory electrical heart failure therapy when necessary. Exemplary devices are known in the art, for example in US6463324, which its contents are incorporated herein by reference entirely.

In some embodiments, a patient is brought into a Cath-Lab, where the device is implanted. In some embodiments, usually, the treatment comprises providing chronic non-excitatory electrical heart failure therapy.

In some embodiments, the already implanted device comprises a controller configured to receive one or more indications, and the implanted device is used to provide acute non-excitatory electrical heart failure therapy in one of more cases according to the indications received. In some embodiments, the indications are received by one or more sources, for example by one or more sensors located within the implanted device, by one or more sensors located outside the device, positioned on the body of the patient and configured to transmit data to the controller of the implanted device, and from an external electronic device and/or server. In some embodiments, a dedicated personnel, for example a physician, provides the indication manually using the one or more external electronic devices. In some embodiments, the patient is allowed to begin the activation of the acute non-excitatory electrical heart failure therapy manually. In some embodiments, the doctor begins the activation of the acute non-excitatory electrical heart failure therapy manually. In some embodiments, a sensor in the device is automatically activated when certain parameters are reached (see below).

### Exemplary parameters of activation of acute non-excitatory electrical heart failure therapy

In some embodiments, an acute non-excitatory electrical heart failure therapy comprises five hours of stimulation per day, a pulse amplitude of 7.5V, a pulse width 5mS and two bipolar pulses per cardiac cycle. In some embodiments, when described herein "providing acute non-excitatory electrical heart failure therapy" it should be understood that it refers to activation using these parameters, unless specifically mentioned differently. In some embodiments, an acute non-excitatory electrical heart failure therapy comprises an increase in one or more parameters related to the therapy. For example, increasing a total of number of pulses provided per day an increment from about 5% to about 200%, increasing a total of number of pulses provided per hour an increment from about 5% to about 200%, increasing a pulse amplitude of pulses provided an increment from about 5% to about 200%, increasing a pulse width of pulses provided an increment from about 5% to about 200%, increasing a number of pulses provided per cardiac cycle an increment from about 5% to about 200%. In some embodiments, the increasing is performed automatically by an acute non-excitatory electrical heart failure device. In some embodiments, the increasing is performed manually by said patient. In some embodiments, after providing therapy the system and/or the patient and/or a dedicated personnel evaluates the status of the patient and/or evaluates an improvement in the condition of said patient, and, when relevant, an amendment in the effective amount of non-excitatory electrical heart failure therapy is performed accordingly.

### Exemplary events triggering acute non-excitatory electrical heart failure therapy

In some embodiments, acute non-excitatory electrical heart failure therapy is provided to a patient on or more of the following events and/or after receiving an indication that one or more of the following events are/had occurred:
1. During an attack of acute angina;
2. After a patient suffered Myocardial Infraction (MI) and/or heart surgery;
3. When a patient shows a positive physiological response to acute non-excitatory electrical heart failure therapy;
4. When a patient requires treatment to improve the performance of daily actions; and
5. When a patient shows a change in his/her condition.

### Exemplary activation of acute non-excitatory electrical heart failure therapy during acute angina

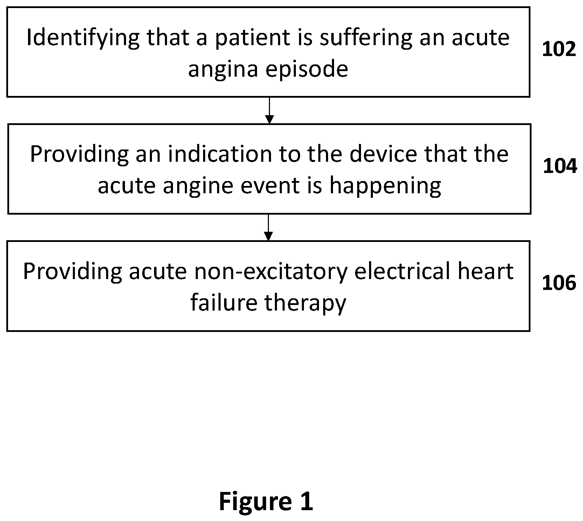

Referring now to Figure 1, showing a flowchart of an exemplary method of treatment of a patient suffering an acute angina episode with acute non-excitatory electrical heart failure therapy, according to some embodiments of the invention. In some embodiments, during an acute angina, the patient and/or the physician may activate the device manually. For example, a patient with history of Angina pectoris that suffers from elevated symptoms of Angina **102,** including for example elevated chest pain, pain in the arms, neck, jaw, shoulder or back, dizziness, fatigue, nausea, shortness of breath and sweating. In some embodiments, during such acute event, the patient and/or physician may trigger acute non-excitatory electrical heart failure therapy, for example by providing an indication to the implanted device that the angina event is happening **104,** for a set period of time from the moment the patient/physician has manually triggered the device **106.** For example, such period can be at least 1min. In some embodiments, such period is typically 5 hours.

In some embodiments, the indication of the angina event is provided automatically to the implanted device by the one or more sensors. For example, data received from the one or more sensors is sent to the controller within the implanted device. In some embodiments, the controller will utilize the data received to translate it into the fact that the angina event is occurring according to predetermined parameters provided to the implanted device.

In some embodiments, in a patient receiving chronic non-excitatory electrical heart failure therapy, an increase in the daily dose of non-excitatory electrical heart failure therapy, such increase can be for example by at least 10%, 20%, 50%, or from about 10% to about 90%. Optionally more than 100% increment.

In some embodiments, in a patient receiving chronic non-excitatory electrical heart failure therapy, provide the daily dose (for example, typically 5 hours per day) during the time of the symptom elevation. Meaning, the "acute" dose is provided as part of the "chronic" dose.

In some embodiments, in a patient receiving chronic non-excitatory electrical heart failure therapy, an increase stimulation parameters, including current amplitude, pulse duration, number of pulses per cardiac cycle will be performed either during provision of an acute or a chronic dose of non-excitatory electrical heart failure therapy. In some embodiments, as mentioned before, exemplary standard stimulation parameters are pulse amplitude of 7.5V, pulse width 5mS and 2 bipolar pulses per cardiac cycle.

In some embodiments, a potential advantage of providing an acute non-excitatory electrical heart failure therapy during an acute angina event is that it will potentially reduce the angina symptoms within a short period of time, for example within minutes or hours.

In some embodiments, the acute non-excitatory electrical heart failure therapy is triggered, for example, by providing a dedicated external device or magnet that, by turning it on or placing it in the proximity of the implantable non-excitatory electrical heart failure therapy device, will activate the acute non-excitatory electrical heart failure therapy.

### Exemplary activation of acute non-excitatory electrical heart failure therapy Post MI and/or Heart surgery

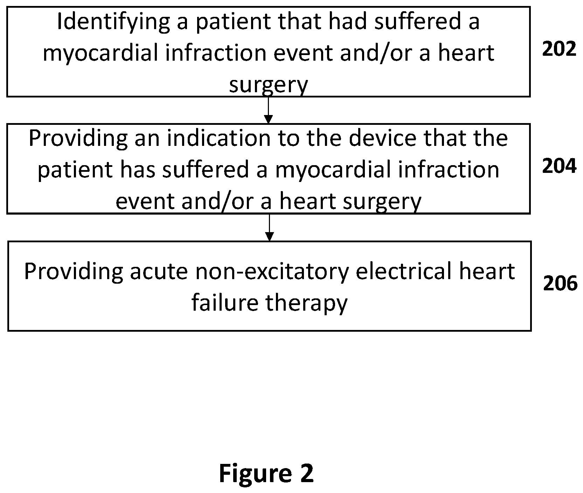

Referring now to Figure 2, showing a flowchart of an exemplary method of treatment of a patient with acute non-excitatory electrical heart failure therapy after suffering a myocardial infraction and/or heart surgery, according to some embodiments of the invention. In some embodiments, after a MI and/or after heart surgery **202,** the patient and/or the physician may activate the device manually **206.** In some embodiments, after a MI and/or after heart surgery **202,** the implanted device is provided with an indication that the patient has suffered a MI and/or after heart surgery **204.** In some embodiments, the device is configured to be automatically activated, either at predetermined periods of time and/or after sensing a predetermined physiological threshold level.

In some embodiments, the activation of the treatment is done automatically in view of data received by the one or more sensors. For example, data received from the one or more sensors is sent to the controller within the implanted device. In some embodiments, the controller will utilize the data received to translate it into the fact that activation of the treatment is required according to predetermined parameters provided to the implanted device.

In some embodiments, exemplary eligible patients are patients having a MI event and/or heart surgery during the last 3 months, preferably during the last 1 month. In some embodiments, such patients will receive an implantable non-excitatory electrical heart failure therapy device that will provide treatment helping the heart to recover from the MI event and/or the surgery event.

In some embodiments, as mentioned before, activation of non-excitatory electrical heart failure therapy comprises 5 hours of stimulation per day, pulse amplitude of 7.5V, pulse width 5mS and 2 bipolar pulses per cardiac cycle.

In some embodiments, a potential advantage of providing an acute non-excitatory electrical heart failure therapy after a MI and/or after heart surgery is that it will potentially provide an improvement of heart parameters, will potentially induce remodeling of damaged tissues, will potentially prevent heart deterioration (which typically happens following MI). In some embodiments, the heart parameters expected to be improved are one or more of: cardiac output, end diastolic volume, valve performances and mechanical performances of the cardiac tissue.

In some embodiments, the device is activated by an operator and continues to deliver therapy for a set period of time, for example, 3 months.

### Exemplary activation of acute non-excitatory electrical heart failure therapy when a patient shows a positive physiological response to acute non-excitatory electrical heart failure therapy - Patient selection

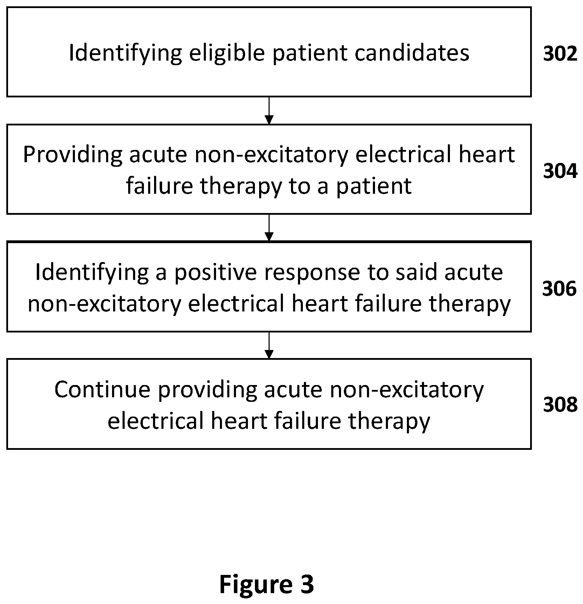

Referring now to Figure 3, showing a flowchart of an exemplary method of treatment of a patient and/or a method of patient selection for treatment with acute non-excitatory electrical heart failure therapy after said patient has shown positive physiological responses to acute non-excitatory electrical heart failure therapy, according to some embodiments of the invention. In some embodiments, first, identification of eligible candidates is performed **302.** In some embodiments, exemplary patients that are eligible for this type of exemplary activation are one or more of:
1. Patients that suffered heart failure (HF) and they are in the Cath-Lab table already, so an acute activation is performed to assess positive feedback from the patient.
2. Any patient in need of improvement in blood flow.
3. Patients that already have an implanted non-excitatory electrical heart failure therapy device.

In some embodiments, after the eligible patient has been identified **302,** the patient is provided with acute non-excitatory electrical heart failure therapy **304** to assess his responses to the therapy **306.** In some embodiments, if the patient responds positively to the therapy, then the patient will continue to receive acute non-excitatory electrical heart failure therapy **308** (thereby positively selecting a patient for receiving acute non-excitatory electrical heart failure therapy).

In some embodiments, exemplary assessments of positive physiological responses to acute non-excitatory electrical heart failure therapy can be one or more of: assessing the change in stroke volume (SV) or Cardiac output (CO), assessing the change of end-diastolic volume (EDV), assessing the change in cardiac contractility that is measured by calculating the stroke volume divided by end-diastolic volume (SV/EDV), assessing the change in cardiac tissue mechanical mobility and assessing the flow rate in the coronary arteries.

In some embodiments, activation of acute non-excitatory electrical heart failure therapy when a patient shows a positive physiological response to acute non-excitatory electrical heart failure therapy comprises one or more of the following actions:
Providing means for non-excitatory electrical heart failure stimulation of a subject. In some embodiments, such means can be an implantable non-excitatory electrical heart failure device or external stimulation device. In some embodiments, in either case, the device is connected to intracardiac electrodes or Epicardiac electrodes (see figure below) that provide non-excitatory electrical heart failure stimulation to the heart.

Performing acute non-excitatory electrical heart failure stimulation testing. In some embodiments, such testing can be done for a set time period (for example a few hours). Optionally, also during non-invasive measurements like echo or angiography. In some embodiments, the stimulation during the test are perform under specific physiological conditions, such as posture conditions, set range of heart rate (HR), etc. In some embodiments, posture can be during laying or sitting. In some embodiments, HR range can be only when HR is below set value, for example 80 BPM. In some embodiments, the tests are done having *on* and *off* periods to assess the response. In some embodiments, it is possible to compare *on* period to *off* periods. In some embodiments, such periods are in the range of seconds to hours, typically few minutes.

Evaluating if there is physiological response of the subject to the acute non-excitatory electrical heart failure therapy testing. In some embodiments, for example, a positive response can be an improvement in the listed parameters above/below of more than 1%, preferably more than 5%, optionally more. In some embodiments, such evaluation is done by comparing the value of selected physiological parameter/s during non-excitatory electrical heart failure therapy period and a non-stimulation period. In some embodiments, as mentioned before, physiological parameters that can show an improvement due to the non-excitatory electrical heart failure therapy and are therefore measured are one or more of: heart rate, heart rate variability, ejection fraction, cardiac output, stroke volume, blood pressure level at the following exemplary locations (arteries, veins, right and left atriums and ventricles, coronaries vein and arteries, carotid arteries and veins and the aorta), cardiac index, vascular resistance, blood oxygen saturation, tissue oxygen saturation, respiratory rate, cardiac impedance, cardiac electro-mechanical timing measurements (for example, pre-ejection period measurement, measurement between electrical activation and specific heart sounds, measurement between electrical activation of the heart and events related to arterial flow, etc.) and seismocardiography (for example, acceleration of heart walls measured by accelerometer sensor on lead).

Identifying if the subject has response to non-excitatory electrical heart failure therapy for the selected physiological parameter/s.

Providing acute non-excitatory electrical heart failure therapy treatment if subject was identified as responsive to non-excitatory electrical heart failure therapy.

### Exemplary activation of acute non-excitatory electrical heart failure therapy when a patient requires treatment to improve the performance of daily actions

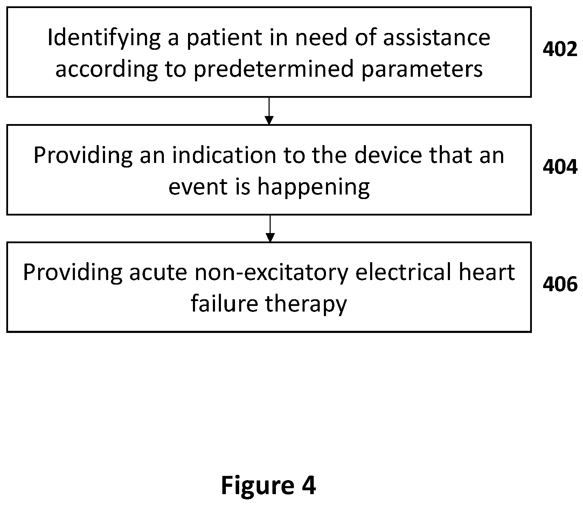

Referring now to Figure 4, showing a flowchart of an exemplary method of treatment of a patient with acute non-excitatory electrical heart failure therapy to assist in performing daily actions, according to some embodiments of the invention. In some embodiments, acute non-excitatory electrical heart failure therapy is provided to improve the quality of life of a patient. In some embodiments, the way acute non-excitatory electrical heart failure therapy improves the quality of life is by helping a patient perform daily tasks by providing the treatment before and/or while performing those tasks. In some embodiments, activation of acute non-excitatory electrical heart failure therapy **406** may be triggered automatically due to a detection of a specific event and/or manually by the subject **402,** for example by providing an indication to the implanted device that the event is happening **404.**

In some embodiments, the activation of the treatment is done automatically in view of data received by the one or more sensors. For example, data received from the one or more sensors is sent to the controller within the implanted device. In some embodiments, the controller will utilize the data received to translate it into the fact that activation of the treatment is required according to predetermined parameters provided to the implanted device.

In some embodiments, for example, events that may require acute non-excitatory electrical heart failure therapy activation can be:
Physical exercise or a social event. In some embodiments, the system comprises a movement sensor (for example, an accelerometer) for measuring the level of exercise and/or movement of the patient. In some embodiments, the system may detect that the movement level is above set value **402** and provide acute non-excitatory electrical heart failure therapy **404** for a set period of time and/or until a value of detected movement returns to another set level value. In some embodiments, set values can be define as absolute values or as relative change from normal level value. For example, the system will provide acute non-excitatory electrical heart failure therapy during exercise (as detected by the movement sensor) and additionally when the measured HR is higher than the average HR of the patient by a set threshold, for example by 20 BPM.

In some embodiments, the activation **404** of the acute non-excitatory electrical heart failure therapy is triggered manually by the patient (See above description on the ways to trigger the treatment).

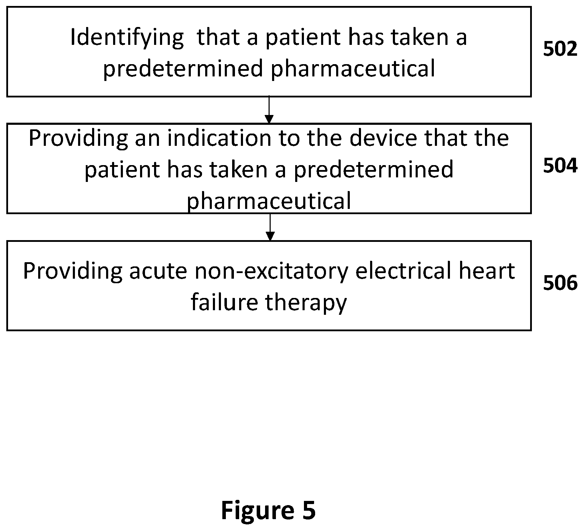

Referring now to Figure 5, showing a flowchart of an exemplary method of treatment of a patient with acute non-excitatory electrical heart failure therapy after said patient has taken a determined pharmaceutical, according to some embodiments of the invention. In some embodiments, the activation **506** of the acute non-excitatory electrical heart failure therapy is triggered by the user, for example by providing an indication to the implanted device that the event is happening **504,** when taking a pharmaceutical **502.** For example, the acute non-excitatory electrical heart failure therapy may triggered manually by the patient following pharmaceutical intake, for example, one or more of the following pharmaceuticals: ACE inhibitors, angiotensin-2 receptor blockers, beta blockers, mineralocorticoid receptor antagonists, diuretics, ivabradine, sacubitril valsartan and hydralazine.

In some embodiments, the system may include a pharmaceutical delivery apparatus that delivers pharmaceuticals at specific time intervals. In some embodiments, the acute non-excitatory electrical heart failure therapy is delivered in synchrony with the timing of the pharmaceutical delivery. For example, before, during or after drug delivery. Additional examples, upon taking an oral drug, the patient triggers the acute non-excitatory electrical heart failure therapy. In some embodiments, the acute non-excitatory electrical heart failure therapy can start, for example, immediately following the triggering event, within 30 min following the triggering event or within 60 min following the triggering event.

In some embodiments, the system may include a sensor that detects the intake of the drug and automatically provides acute non-excitatory electrical heart failure therapy in synchrony with the timing of the drug intake.

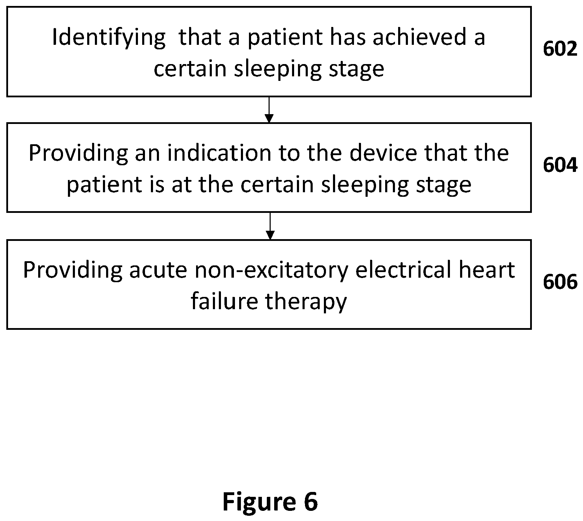

Referring now to Figure 6, showing a flowchart of an exemplary method of treatment of a patient with acute non-excitatory electrical heart failure therapy during sleep, according to some embodiments of the invention. In some embodiments, acute non-excitatory electrical heart failure therapy is provided during sleep. In some embodiments, the system comprises a sleep sensor that evaluates the sleep stages. In some embodiments, the system will provide acute non-excitatory electrical heart failure therapy **606** during a specific sleep stage **602,** for example by providing an indication to the implanted device that the patient has reached the specific sleep stage **604.**

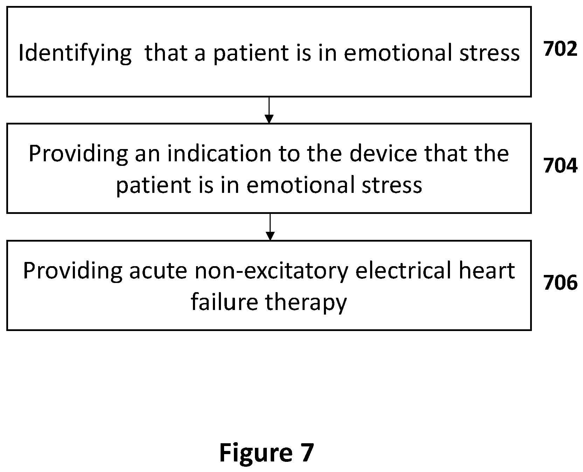

Referring now to Figure 7, showing a flowchart of an exemplary method of treatment of a patient with acute non-excitatory electrical heart failure therapy during emotional stress, according to some embodiments of the invention. In some embodiments, acute non-excitatory electrical heart failure therapy is provided during emotional stress. In some embodiments, emotional stress is identified **702** either by the user, which activates the acute non-excitatory electrical heart failure therapy **706,** for example by providing an indication to the implanted device that the event is happening **704,** and/or by receiving the indication from one or more sensors, for example, a level of sweat sensor (such as GSR) for measuring the level of emotional stress, heart rate sensor, blood pressure sensor, etc. In some embodiments, once the sensors detect a certain level which above a predetermined threshold, the system then activates the acute non-excitatory electrical heart failure therapy.

In some embodiments, emotional stress can happen contemporarily to physical stress. For example, during sexual intercourse, where the male requires better blood flow to achieve a better erection, and/or where the female may benefit from improved blood flow to her relevant organs. Another example, acute non-excitatory electrical heart failure therapy may be activated, for example, as stress as a result of anger or other factors that increase the sympathetic system. In some embodiments, the system may detect that the stress level is above set value and provide acute non-excitatory electrical heart failure therapy for a set period of time and/or until the detected value of stress returns to another set level value.

### Exemplary activation of acute non-excitatory electrical heart failure therapy when a patient shows a change in his/her condition

In some embodiments, as explained herein elsewhere, one or more sensor monitor the status of the patient, for example, a level of sweat sensor (such as GSR) for measuring the level of emotional stress, heart rate sensor, blood pressure sensor, temperature sensor, flow, pressure and/or acceleration sensors. In some embodiments, data from the one or more sensors is analyzed to assess whether a state of a patient has changed (either for better or worse). In some embodiments, from the result of the analysis, the system is configured to utilize that data to assess whether the change in the state of the patient requires intervention, or in other words the data is used to provide an indication to the system that treatment is needed. In some embodiments, intervention can be one or more of: notifying a dedicated medical personnel, amending the acute non-excitatory electrical heart failure therapy treatment, stopping the acute non-excitatory electrical heart failure therapy treatment and commencing the acute non-excitatory electrical heart failure therapy treatment.

For example, from the data from the one or more sensors the system identifies that the patient has an acute response to the acute non-excitatory electrical heart failure therapy, monitored for example by monitoring the left ventricle pressure and/or left ventricle ejection fraction. In some embodiments, this identification is translated into the indication the system needs to perform an action. In some embodiments, for example, in view of this, the system can either stop providing acute non-excitatory electrical heart failure therapy, or can amend the acute non-excitatory electrical heart failure therapy, by amending for example one or more of pulse amplitude, stimulation dose, etc.

In some embodiments, monitoring a change in the state of the patient includes requesting from the patient to perform a test, for example, to perform an exercise (for example walking for 6 minutes) and assessing one or more parameters, for example, left ventricular ejection fraction. In some embodiments, this information is translated into the indication the system needs to perform an action. In some embodiments, for example, based on the results, the acute non-excitatory electrical heart failure therapy can be either stopped or amended, as explained above.

In some embodiments, for example, the temperature sensor monitors the current temperature of the patient, and according to the temperature of the patient and/or a change in the temperature of the patient, the system assesses whether to provide, amend or stop the acute non-excitatory electrical heart failure therapy. For example, if the temperature sensor shows that the patient suffers from a fever, the acute non-excitatory electrical heart failure therapy will not be provided. In some embodiments, the system is configured to provide acute non-excitatory electrical heart failure therapy only when the temperature sensed by the temperature sensor is between a predetermined range, for example, between a normal temperature range, for example between about 35 degrees and 37.5 degrees. In some embodiments, the predetermined range is set by a dedicated personnel. In some embodiments, a predetermined increase in the temperature can cause activating the acute non-excitatory electrical heart failure therapy, for example, an increase of from about 0.5 degrees to about 1 degrees can cause the activation of the acute non-excitatory electrical heart failure therapy.

### Exemplary cases for activation or non-activation of acute non-excitatory electrical heart failure therapy according to exemplary cases

In some embodiments, depending on the case and the physiological state of that subject, the device is configured to either provide acute non-excitatory electrical heart failure therapy or not. In some embodiments, the main reason of not providing acute non-excitatory electrical heart failure therapy is that activation of the device would not contribute to the state of the subject and/or would potentially cause damage to the subject, or at least, receiving an indication that providing treatment would not provide the desired effect.

The following table includes exemplary cases (exemplary physiological states/exemplary indications provided to the system) where the acute non-excitatory electrical heart failure therapy is either provided or not, it should be understood that the following table provides examples in order to allow a person having skills in the art to understand the invention and are not meant to limit the invention in any case:

| Physiological state of patient | Effect of providing acute non-excitatory electrical heart failure therapy | To provide or not to provide |
|---|---|---|
| Tired | Increase in blood flow | Yes |
| Angry | | No |
| Sleepiness | | Yes |
| Reduced oxygen saturation level | | Yes |
| Insomnia | | No |
| Muscle weakness | | Yes |

### Exemplary devices and systems for the treatment

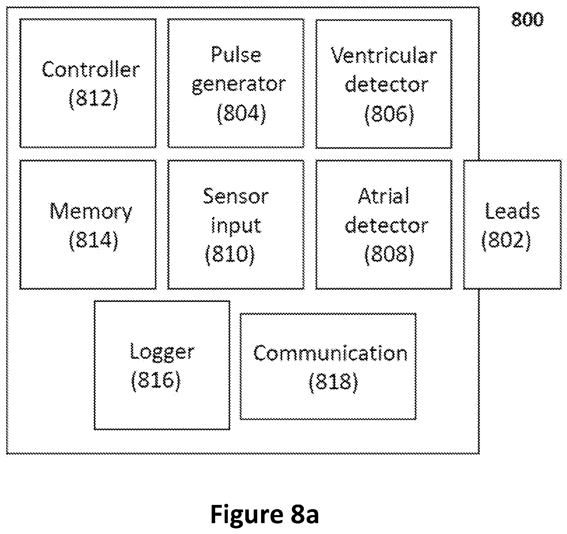

Figure 8a is a schematic block diagram of an exemplary cardiac therapy device **800,** in accordance with some embodiments of the invention.

Device **800,** as shown, includes one or more leads **802** (optionally two leads), which are optionally couplable to device **800** at one or more can connectors (not shown).

A pulse generator **804** is optionally used to generate the signal, for example, including a power circuitry, for example, including one or more storage capacitors.

In some embodiments of the invention, a ventricular detector **806** is provided and used to detect atypical ventricular activation, which can be a contra-indication to signal application.

In some embodiments of the invention, an atrial detector **808** is provided and used to detect atypical atrial activation, which may be used as an input to decision making by device **800.**

A sensor input **810** may receive data from one or more sensors, for example electrical sensors or other sensors, such as flow, pressure and/or acceleration sensors. Data from the sensors is optionally further processed (e.g., by a controller **812** and/or detectors **806, 808**) and are optionally be used as an input to decision making processes in device **800.**

A controller **812** is optionally provided and executes one or more logics to decide, for example, a timing and/or other parameters of a signal and/or if a signal is to be applied.

In some embodiments, the controller controls the applying of stimulation pulses according to the treatment plan. Optionally, the controller effects a change in the plan, for example so as to compensate for real time deviations from the treatment plan (e.g. a skipped stimulation).

A memory **814** is optionally provided, for example, to store logic, past effects, therapeutic plan, adverse events and/or pulse parameters.

In some embodiments, the controller and/or memory are programmed with one or more treatment plans (optionally set for the specific patient) and/or with one or more fallback treatment plans.

In some embodiments, instructions for "compensating" for a change from the planned treatment are stored and are addressed by the device controller when relevant, including for example modifications in treatment duration, number of stimulations, stimulation signal parameters (e.g. current intensity), and/or other modifications which may be applied to compensate for a real time change in the original plan. In some embodiments, the instructions include numerical factors according to which one or more parameters of treatment are modified. In some examples, the instructions may include: a factor by which the stimulation current intensity should be multiplied in case the actual applied stimulations did not reach the target amount of energy; a factor by which output voltage should be modulated to result in the desired current intensity; a factor by which the stimulation rate should be multiplied in case the actual applied stimulation did not reach the target total number of stimulations (optionally within a set time period); time related modifications such as factors by which the treatment session duration should be lengthened in case a target was not reached; and the like.

In some embodiments, the controller refers to a look up table or the like which ties between specific situations (e.g. a skipped stimulation, number of actual stimulations delivered being lower than planned, etc.) and the instructions for compensating for that situation (e.g. by updating one or more parameters, such as updating the length of a treatment session).

A logger **816** is optionally provided to store activities of device **800** and/or of the patient. Such a log and/or programming may use a communication module **818** (e.g., of a type known in the art) to send data from device **800,** for example, to a programmer (not shown) and/or to receive data, for example, programming, for example, pulse parameters.

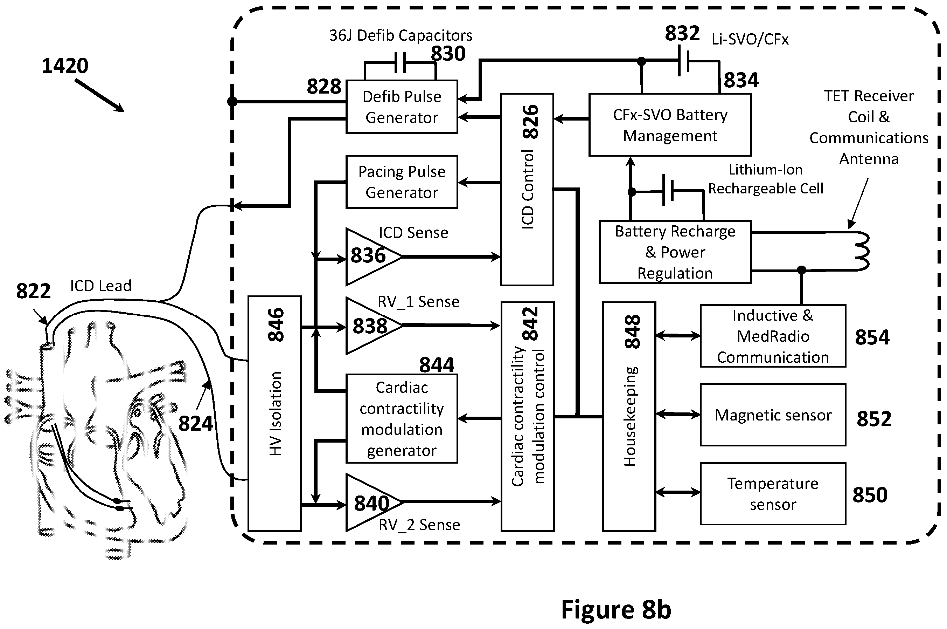

FIG. 8b is a schematic diagram showing components of an implantable cardiac device **820,** according to some embodiments.

In some embodiments, the device is configured for delivery of cardiac electrical stimulation, such as cardiac contractility modulation stimulation. Optionally, the device is further configured to function as cardioverter defibrillator (ICD).

In some embodiments, the device comprises an ICD lead **822,** and a cardiac contractility modulation lead **824.**

In some embodiments, activation of the ICD lead is by an ICD module which includes or is connected to: ICD control **826,** a defibrillation pulse generator **828** (via one or more capacitors **830**), a power source (e.g. a battery **832**) and power source management circuitry **834,** and ICD sense **836** which senses an applied pulse to verify the pulse is within a selected (e.g. programmed) amplitude and/or duration. In some embodiments, activation of the one or more cardiac contractility modulation leads **838, 840,** which are optionally positioned in the right ventricle, is by a cardiac contractility modulation module which includes or is connected to: cardiac contractility modulation control **842,** a cardiac contractility modulation generator **844.**

It is noted that in some embodiments, the ICD coil and one more electrodes for pacing and/or cardiac contractility modulation are configured on the same lead.

In some embodiments, the leads are connected to isolation **846.**

In some embodiments, the device comprises a housekeeping module **848,** which includes or is connected to one or more sensor such as a temperature sensor **850,** a magnetic sensor **852,** and communication means **854** such as an antennae, a receiver and the like. Other sensors may include flow sensors, pressure sensors, acceleration sensors, and/or other.

In some embodiments, data received from the one or more sensors is received as input. Optionally, the input is processed by the device control (e.g. by the ICD control, cardiac contractility modulation control, and/or a general controller, not shown) and is optionally used as input to decision making processes in device **820.**

In some embodiments, the device control (e.g. the ICD control, cardiac contractility modulation control, and/or a general controller, not shown) executes one or more logics to decide, for example, a timing and/or other parameters of a signal and/or if a signal is to be applied.

A memory (not shown) is optionally provided, for example, to store logic, past effects, therapeutic plan, adverse events and/or pulse parameters.

A logger (not shown) is optionally provided to store activities of device **820** and/or of the patient. Such a log and/or programming may use a communication module **854** to send data from device **820,** for example, to a programmer (not shown) and/or to receive data, for example, programming, for example, pulse parameters.

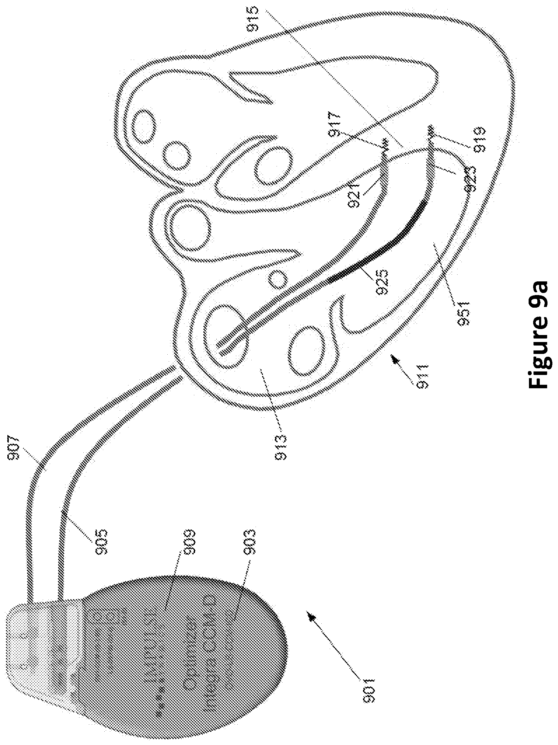

Figure 9a schematically illustrates an exemplary implanted device configured for applying cardiac electrical stimulation, according to some embodiments of the invention.

In some embodiments, implantable device **901** comprises a pulse generator **903.** In some embodiments, pulse generator **903** comprises a housing **909** which encases, for example: powering means (e.g. a battery), control circuitry (e.g. a controller) configured for timing and generating the electrical pulses, sensing circuitry, communication circuitry, memory means, and/or other operational modules.

In some embodiments, one or more stimulation leads such as **905, 907** are connected to the housing and extend externally from it. In some embodiments, a lead comprises one or more electrical wires, surrounded by an external insulating layer. In some embodiments, a lead is comprised of two wires, having different polarities. In some embodiments, a wire of the lead is coiled.

In some embodiments, pulse generator **903** is implanted outside the heart, for example in the subclavian area. Optionally, implantation is via a minimally invasive procedure.

In some embodiments, a housing of pulse generator **903** is implanted subcutaneously, in proximity of the left chest.

In some embodiments, leads **905** and **907** extend from pulse generator **903,** and at least a distal segment of the leads is implanted within the heart **911.** In some embodiments, as shown, both leads are passed through the right atrium **913,** and contact, at their distal ends, the ventricular septum **915.** In some embodiments, each lead contacts the septum at a different location.

It is noted that additionally or alternatively, a single lead which includes two spaced apart stimulation electrodes may be used.

It is also noted that while the figures shows both leads being positioned in the right ventricle **931** against ventricular septum **915,** one or more stimulating leads may be in other locations, with consequently different effect circles and/or targeting different tissues. In some embodiments, the leads are located inside the heart, on the right side thereof optionally to take advantage of two potential advantages: a. less out-of-heart tissue being stimulated; and b. less invasive access and/or presence than in the left heart.

In some embodiments, one of the leads is implanted outside the heart, and the other lead is implanted inside the heart.

In some embodiments, each of the leads ends with a tip electrode (see **917** of lead **907, 919** of lead **905**). The tip electrode may be configured as a contact electrode, a screw-in electrode, a sutured electrode, a free-floating electrode and/or other types.

In some embodiments, one or both of the leads includes a ring electrode (see **921** of lead **907, 923** of lead **905**), located along the lead, proximally to the tip electrode.

In some embodiments, the electrodes are implanted in the right ventricle or in the right atria of the heart.

In some embodiments, a tip electrode is formed with a threading so as to be threaded into the tissue. Alternatively, a tip electrode is solely placed in contact with the tissue.

In some embodiments, one or both of the leads includes a defibrillation coil (see **925** of lead **905**). Optionally, coil **925** is located along the lead, proximally to the tip electrode and/or proximally to the ring electrode.

In some embodiments, the coil is implanted in the right ventricle, right atria or in the vena cava.

In some embodiments, one or both leads are configured to deliver a non-excitatory signal such as a cardiac contractility modulation signal.

In some embodiments, the cardiac contractility modulation signal is applied in contact with the ventricular tissue or within ventricular tissue.

In some embodiments, the cardiac contractility modulation signal is applied to the heart during a relative and/or absolute refractory period of the heart. In some embodiments, the signal is selected to increase the contractility of a cardiac ventricle when the electric field of the signal stimulates such ventricular tissue, for example, the left ventricle, the right ventricle and/or a ventricular septum. In some embodiments of the invention, contractility modulation is provided by phosphorylation of phospholamban caused by the signal. In some embodiments of the invention, contractility modulation is caused by a change in protein transcription and/or mRNA creation caused by the signal, optionally in the form of reversal of a fetal gene program. It is noted that in some embodiments the cardiac contractility modulation signal may be excitatory to tissue other than that to which it is applied.

While not being limited to a single pulse sequence, the term cardiac contractility modulation is used to describe any of a family of signals which includes a significant component applied during an absolute refractory period and which has a clinically significant effect on cardiac contractility in an acute and/or chronic fashion and/or which causes a reversal of fetal gene programs and/or which increases phosphorylation of phospholamban. In some embodiments, the signal is potentially excitatory to one part of the heart but non-excitatory to other parts. For example, a signal can be excitatory in atria, but applied at a timing (relative to ventricular activation) when it is not excitatory in the ventricle.

In some embodiments of the invention, the signal while potentially stimulatory during the receptive period of the cardiac cycle, is non-excitatory due to its timing. In particular, the signal is applied during the refractory period of the tissue which is affected by it and, optionally, within the absolute refractory period.

In some embodiment, a device electrode, for example the cardiac contractility modulation applying electrode, is used for measuring the R wave amplitude and/or RR interval of the cardiac cycle.

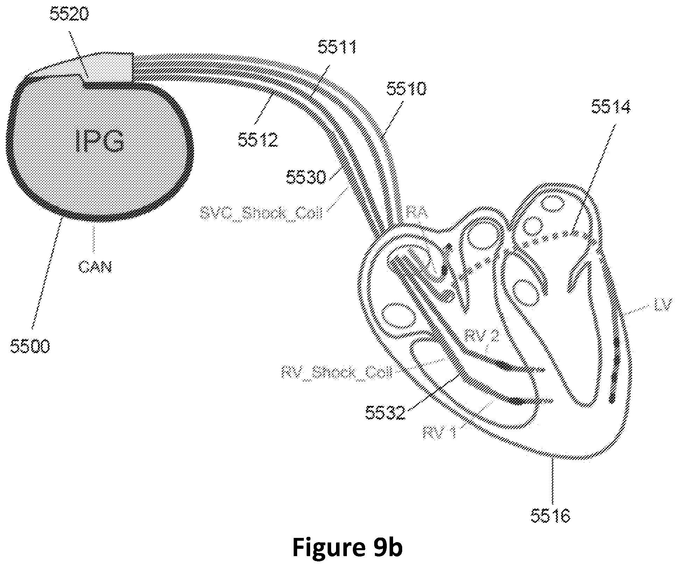

Figure 9b schematically illustrates an exemplary cardiac device comprising a plurality of leads, according to some embodiments of the invention. In some embodiments, device **5500** is configured for delivering cardiac contractility modulation stimulations to the heart. In some embodiments, device **5500** is further configured to function as a cardiac defibrillator (ICD).

In some embodiments, device **5500** comprises a plurality of leads. In the example shown, a first lead **5510** extends to the right atrium of the heart **5516;** a second lead **5511,** such as for applying cardiac contractility modulation stimulation, extends to the right ventricle, optionally contacting the ventricular septum; a third lead **5512** extends to the right ventricle, optionally contacting the ventricular septum; and a fourth lead **5514** extending to the left ventricle, for example through the coronary sinus. In some embodiments, one or more of the leads comprises a defibrillation coil. In this example, lead **5512** comprises a superior-vena cava shock coil **5530** and a right ventricle shock coil **5532.**

In some embodiments, the plurality of leads are connected to the device housing **5520** via a plurality of ports (not shown). In some embodiments, control of activation of one or more leads is via switch circuitry, for example switch circuitry of the device controller.

### Exemplary General information about planning and devices

In some embodiments, the method for cardiac electrical stimulation, comprises: defining a treatment plan including parameters according to which cardiac electrical stimulations are applied to the heart; applying cardiac electrical stimulations to the heart according to the treatment plan; during applying, adapting the treatment to actual cardiac activity; and automatically updating at least one of the parameters to compensate for any changes made relative to the treatment plan due to the adapting.

In some embodiments, the parameters include one or more of: a rate of cardiac electrical stimulations; a time period over which cardiac electrical stimulations are to be applied; stimulation current; an output voltage; a duration of each stimulation.

In some embodiments, defining a treatment plan comprises selecting the parameters so as to deliver one or both of: a total number of cardiac electrical stimulations; a total amount of cardiac electrical stimulation energy.

In some embodiments, automatically updating comprises one or more of: increasing or reducing the rate of cardiac electrical stimulations; lengthening or shortening the time period over which cardiac electrical stimulations are applied; increasing or reducing the stimulation current intensity; lengthening or shortening the stimulation duration.

In some embodiments, defining a treatment plan comprises measuring or receiving input of cardiac activity characteristics of a patient being treated.

In some embodiments, the cardiac activity characteristics include: average heart rate, average stroke volume, occurrence rate of irregular cardiac events.

In some embodiments, the irregular cardiac events are from the group of: premature ventricular contraction (PVC), atrial arrhythmia or ventricle arrhythmia.

In some embodiments, applying comprises delivering an electrical stimulation via an implanted device comprising one or more leads which contact the ventricular septum of the heart.

In some embodiments, the method comprises measuring the actual cardiac activity using one or more sensors.

In some embodiments, defining a treatment plan comprises setting one or more thresholds of a heart rate during which the cardiac electrical stimulations are to be applied. For example, when the HR is from about 5% to about 10% higher than a predetermined value, acute non-excitatory electrical heart failure therapy is provided.

In some embodiments, defining a treatment plan comprises selecting one or more physical states of the patient during which the cardiac electrical stimulations are to be applied.

In some embodiments, automatically updating is performed in response to one or more skipped stimulations.

In some embodiments, automatically updating is performed if a number of cardiac electrical stimulations that were actually delivered is lower than a planned number of cardiac electrical stimulations.

In some embodiments, automatically updating is performed if a total amount of cardiac electrical stimulation energy delivered is lower than a planned amount of cardiac electrical stimulation energy.

In some embodiments, the cardiac electrical stimulation comprises cardiac contractility modulation stimulation.

In some embodiments, the method for delivering a therapeutic stimulation to the heart, comprises: selecting a total number of stimulations to be delivered to the heart over a selected period of time; delivering stimulations to the heart; counting the number of stimulations actually delivered; if the number of stimulations actually delivered is lower than the selected total number of stimulations, adding a selected time period at an extent sufficient to deliver the required additional stimulations.

In some embodiments, the stimulations comprise cardiac contractility modulation stimulations.

In some embodiments, the method comprises adding the selected time period to deliver additional stimulations if the number of stimulations actually delivered is lower than 90% of the selected total number of stimulations.

In some embodiments, adding a selected time period comprises extending the period of time that was initially selected.

In some embodiments, if the number of stimulations actually delivered is lower than the selected total number of cardiac contractility modulation stimulations, reducing the selected total number of cardiac contractility modulation stimulations for a future stimulation session.

In some embodiments, the selected time period is between 4 hours - 8 hours a day.

In some embodiments, the method for planning cardiac electrical stimulation treatment, comprises: selecting a total amount of energy to be delivered by cardiac electrical stimulations to the heart; and selecting one or both of: a time period during which cardiac electrical stimulations are to be applied to the heart, and stimulation current intensity for each of the stimulations, the time period and the stimulation current intensity selected so as to reach the total amount of energy.

In some embodiments, the cardiac electrical stimulation comprises cardiac contractility modulation stimulation.

In some embodiments, the system for cardiac electrical stimulation treatment, comprises: an implantable pulse generator; one or more leads extending from the pulse generator to the heart for applying cardiac electrical stimulation; a controller programmed with at least one treatment plan for applying cardiac electrical stimulations, the controller configured to automatically update the treatment plan in response to actual cardiac activity by updating one or more of: a time period during which cardiac electrical stimulations are applied; a rate of cardiac electrical stimulations; an amount of energy delivered at each cardiac electrical stimulation.

In some embodiments, the system comprises one or more sensors including an ECG sensor configured for measuring the actual cardiac activity.

In some embodiments, one or more leads contact the ventricular septum of the heart.

In some embodiments, the controller is programmed with instructions for automatically updating the treatment plan, the instructions suitable to compensate for real time changes in the treatment plan.

In some embodiments, the instructions include numerical factors according to which one or more of the following parameters are updated: stimulation current intensity; output voltage which sets a selected stimulation current intensity; stimulation duration; treatment session duration; stimulation rate.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

In some embodiments, planning and delivering cardiac electrical stimulation treatment are performed and, more particularly, but not exclusively, cardiac electrical stimulation treatment which parameters can be updated based on and/or in response to actual cardiac activity are performed.

In some embodiments, the stimulation treatment is planned according to characteristics of cardiac activity and is optionally modified in view of actual cardiac activity. In some embodiments, stimulation is selected and/or performed taking into account a natural variability in the function of the heart, including both intra-person and inter-person variability.

In some embodiments, updating one or more parameters of cardiac electrical stimulation treatment, optionally in real time, is performed to compensate for any changes made in the treatment due to actual cardiac activity.

In some embodiments, a treatment plan is defined by setting parameters such as: stimulation rate, stimulation current intensity, a duration of a treatment session, and/or other parameters. In some embodiments, the parameters are selected according to a general treatment goal, for example, a total number of cardiac electrical stimulations to be delivered to the heart, and/or a total amount of cardiac electrical stimulation energy to be delivered to the heart.

In some embodiments, the treatment plan is defined according to characteristics of cardiac activity of the patient being treated. For example, taking into account characteristics such as the patient's heart rate, stroke volume, occurrence rate of irregular cardiac events (such as arrhythmia), and/or other characteristics.

In some embodiments, the treatment plan defines one or more conditions for applying the cardiac electrical stimulation. For example, the plan defines that stimulation should be delivered only when the heart rate is lower and/or higher than a threshold (or within a selected range). For example, the plan defines that stimulation should be delivered only in certain physical states of the patient, such as only during rest.

In some embodiments, the treatment plan takes into account expected variability in cardiac activity. In some embodiments, treatment parameters such as the total number of stimulations to be delivered; the total amount of stimulation energy to be delivered; the rate of stimulating; the timing of stimulation are selected taking into account that a change in a parameter may have an effect that is non-linear on the treatment itself. Therefore, in some embodiments, multiple sets of parameters may be defined for obtaining equivalent treatment effects. For example, it may be that delivery of 8000 stimulations randomly dispersed over a day would obtain a treatment effect which is equivalent to stimulations delivered at each cardiac beat for one hour at a heart rate higher than 90 bpm. In some embodiments, systems and/or devices for example as described herein (e.g. the system controller) are preprogrammed with a look-up table including parameter sets, which optionally lead to equivalent treatment effects and may be interchanged. In some embodiments, a treatment parameter is selected and/or calculated according to one more additional treatment parameters and/or according to a desired treatment effect.

In some embodiments, cardiac electrical stimulation treatment is delivered according to the plan, but variations may be made in the plan in response to actual cardiac activity. Optionally, variations are made in real time. For example, if an irregular beat is identified, stimulation at that beat may be skipped. In some embodiments, variations in the treatment plan are made in response to actual cardiac activity measured (and optionally monitored over time) by one or more sensors, such as via ECG measurement.

In some embodiments, the variations made in the plan are compensated for, for example so as to reach the general treatment goal. In some embodiments, compensating comprises updating one or more treatment parameters, such as: updating a duration of a treatment session; updating the rate of cardiac electrical stimulations; updating the current intensity of the stimulations; updating the number of electrodes being activated (for example so as to contact a different sized area of tissue); updating the time interval and/or number of beats between consecutive stimulations; and/or other treatment parameters.

In some embodiments, an implantable device is provided, including one or more leads for delivering the cardiac electrical stimulation and a controller configured for controlling stimulation via the leads. In some embodiments, the controller is configured for automatically updating one or more treatment parameters in response to a variation made in the treatment plan due to actual cardiac activity. In some embodiments, the controller is programmed with one or more treatment plans and one or more "fallback" instructions for updating the treatment parameters when treatment is being carried out and is optionally changed due to actual cardiac activity.

Some examples of "fallback" instructions include: lengthening a treatment session duration if the number of stimulations actually delivered is smaller than a set number of stimulations; increasing an intensity of the stimulation current if the total amount of energy delivered is lower than a set amount of energy to be delivered; increasing the rate of cardiac electrical stimulations if a treatment session time period has almost ended but not enough stimulations were actually delivered; updating a heart rate threshold for applying of stimulations if the actual heart rate over the treatment session (or a part of it) was not within the defined heart rate for delivery of stimulations.

As used herein with reference to quantity or value, the term "about" means "within ± 20 % of".

The terms "comprises", "comprising", "includes", "including", "has", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular forms "a", "an" and "the" include plural references unless the context clearly dictates otherwise. For example, the term "a compound" or "at least one compound" may include a plurality of compounds, including mixtures thereof.

Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Unless otherwise indicated, numbers used herein and any number ranges based thereon are approximations within the accuracy of reasonable measurement and rounding errors as understood by persons skilled in the art.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

It is the intent of the applicant(s) that all publications, patents and patent applications referred to in this specification are to be incorporated in their entirety by reference into the specification, as if each individual publication, patent or patent application was specifically and individually noted when referenced that it is to be incorporated herein by reference. In addition, citation or identification of any reference in this application shall not be construed as an admission that such reference is available as prior art to the present invention. To the extent that section headings are used, they should not be construed as necessarily limiting. In addition, any priority document(s) of this application is/are hereby incorporated herein by reference in its/their entirety.
The present disclosure further relates to the following aspects:
Aspect 1: A system for cardiac stimulation, comprising an implantable controller, said controller configured to carry out the method of:
   a. receiving an indication that a patient is suffering of an acute angina event;
   b. providing an effective amount of non-excitatory electrical heart failure therapy.
Aspect 2: The system according to Aspect 1, wherein said system further comprises at least one sensor configured for detecting said acute angina event; and wherein said indication is received from said at least one sensor.
Aspect 3: The system according to Aspect 2, wherein said at least one sensor is located within said system and implanted within a body of said patient.
Aspect 4: The system according to Aspect 2, wherein said at least one sensor is located outside a body of said patient and wherein said indication is transmitted to said implantable controller.
Aspect 5: The system according to Aspect 1, wherein said indication is provided to said implantable controller by an external source.
Aspect 6: The system according to Aspect 5, wherein said external source is one or more of a physician, said patient and an external device.
Aspect 7: The system according to Aspect 1, wherein said effective amount of non-excitatory electrical heart failure therapy comprises providing a pulse amplitude of 7.5V, a pulse width 5mS and two bipolar pulses per cardiac cycle.
Aspect 8: The system according to Aspect 1, wherein said providing comprises one or more of:
   a. providing said effective amount of non-excitatory electrical heart failure therapy for at least 5 hours of stimulation per day;
   b. increasing a total of number of pulses provided per day an increment from about 5% to about 200%;
   c. increasing a total of number of pulses provided per hour an increment from about 5% to about 200%;
   d. increasing a pulse amplitude of pulses provided an increment from about 5% to about 200%;
   e. increasing a pulse width of pulses provided an increment from about 5% to about 200%;
   f. increasing a number of pulses provided per cardiac cycle an increment from about 5% to about 200%.
Aspect 9: The system according to Aspect 8, wherein said increasing is performed by one or more of:
   a. automatically by an acute non-excitatory electrical heart failure device;
   b. manually by said patient.
Aspect 10: The system according to Aspect 1, wherein said controller is further configured to receive an indication of an improvement in said acute angina event, and further configured for amending said effective amount of non-excitatory electrical heart failure therapy accordingly.
Aspect 11: A system for cardiac stimulation, comprising an implantable controller, said controller configured to carry out the method of:
   a. receiving an indication that a patient had suffered a myocardial infraction event and/or had heart surgery;
   b. providing an effective amount of non-excitatory electrical heart failure therapy.
Aspect 12: The system according to Aspect 11, wherein said system further comprises at least one sensor configured for detecting said myocardial infraction event; and wherein said indication is received from said at least one sensor.
Aspect 13: The system according to Aspect 12, wherein said at least one sensor is located within said system and implanted within a body of said patient.
Aspect 14: The system according to Aspect 12, wherein said at least one sensor is located outside a body of said patient and wherein said indication is transmitted to said implantable controller.
Aspect 15: The system according to Aspect 11, wherein said indication is provided to said implantable controller by an external source.
Aspect 16: The system according to Aspect 15, wherein said external source is one or more of a physician, said patient and an external device.
Aspect 17: The system according to Aspect 11, wherein said effective amount of non-excitatory electrical heart failure therapy comprises providing a pulse amplitude of 7.5V, a pulse width 5mS and two bipolar pulses per cardiac cycle.
Aspect 18: The system according to Aspect 11, wherein said providing comprises one or more of:
   a. providing said effective amount of non-excitatory electrical heart failure therapy for at least 5 hours of stimulation per day;
   b. increasing a total of number of pulses provided per day an increment from about 5% to about 200%;
   c. increasing a total of number of pulses provided per hour an increment from about 5% to about 200%;
   d. increasing a pulse amplitude of pulses provided an increment from about 5% to about 200%;
   e. increasing a pulse width of pulses provided an increment from about 5% to about 200%;
   f. increasing a number of pulses provided per cardiac cycle an increment from about 5% to about 200%.
Aspect 19: The system according to Aspect 18, wherein said increasing is performed by one or more of:
   a. automatically by an acute non-excitatory electrical heart failure device;
   b. manually by said patient.
Aspect 20: The system according to Aspect 11, wherein said controller is further configured to receive an indication of an improvement in a status of said patient, and further configured for amending said effective amount of non-excitatory electrical heart failure therapy accordingly.
Aspect 21: A method of patient selection for receiving a treatment with acute non-excitatory electrical heart failure therapy, the method comprising:
   a. identifying an eligible patient for acute non-excitatory electrical heart failure therapy;
   b. providing means to perform acute non-excitatory electrical heart failure therapy;
   c. performing test to validate positive responses to acute non-excitatory electrical heart failure therapy by providing acute non-excitatory electrical heart failure therapy to said patient; and
   d. assessing, based on a result of said test, that said patient positively responds to acute non-excitatory electrical heart failure therapy.
Aspect 22: The method according to Aspect 21, further comprising continuing providing an effective amount of non-excitatory electrical heart failure therapy.
Aspect 23: The method according to Aspect 21, wherein said positive responses are positive physiological responses.
Aspect 24: The method according to Aspect 23, wherein said positive physiological responses are one or more of: a change in stroke volume (SV), a change in Cardiac output (CO), a change of end-diastolic volume (EDV), a change in cardiac contractility that is measured by calculating the stroke volume divided by end-diastolic volume (SV/EDV), a change in cardiac tissue mechanical mobility and a flow rate in the coronary arteries.
Aspect 25: The method according to Aspect 22, wherein said effective amount of non-excitatory electrical heart failure therapy comprises providing a pulse amplitude of 7.5V, a pulse width 5mS and two bipolar pulses per cardiac cycle.
Aspect 26: The method according to Aspect 22, wherein said providing comprises one or more of:
   a. providing said effective amount of non-excitatory electrical heart failure therapy for at least 5 hours of stimulation per day;
   b. increasing a total of number of pulses provided per day an increment from about 5% to about 200%;
   c. increasing a total of number of pulses provided per hour an increment from about 5% to about 200%;
   d. increasing a pulse amplitude of pulses provided an increment from about 5% to about 200%;
   e. increasing a pulse width of pulses provided an increment from about 5% to about 200%;
   f. increasing a number of pulses provided per cardiac cycle an increment from about 5% to about 200%.
Aspect 27: The method according to Aspect 26, wherein said increasing is performed by one or more of:
   a. automatically by an acute non-excitatory electrical heart failure device;
   b. manually by said patient.
Aspect 28: The method according to Aspect 22, further comprising continuously evaluating positive responds in said patient, and further comprising amending said effective amount of non-excitatory electrical heart failure therapy accordingly.
Aspect 29: A system for cardiac stimulation, comprising an implantable controller, said controller configured to carry out the method of:
   a. receiving an indication that a patient is in need of an increase in blood flow;
   b. providing an effective amount of non-excitatory electrical heart failure therapy.
Aspect 30: The system according to Aspect 29, wherein said system further comprises at least one sensor configured for detecting said need of an increase in blood flow; and wherein said indication is received from said at least one sensor.
Aspect 31: The system according to Aspect 30, wherein said at least one sensor is located within said system and implanted within a body of said patient.
Aspect 32: The system according to Aspect 30, wherein said at least one sensor is located outside a body of said patient and wherein said indication is transmitted to said implantable controller.
Aspect 33: The system according to Aspect 29, wherein said indication is provided to said implantable controller by an external source.
Aspect 34: The system according to Aspect 33, wherein said external source is one or more of a physician, said patient and an external device.
Aspect 35: The system according to Aspect 29, wherein said effective amount of non-excitatory electrical heart failure therapy comprises providing a pulse amplitude of 7.5V, a pulse width 5mS and two bipolar pulses per cardiac cycle.
Aspect 36: The system according to Aspect 29, wherein said providing comprises one or more of:
   a. providing said effective amount of non-excitatory electrical heart failure therapy for at least 5 hours of stimulation per day;
   b. increasing a total of number of pulses provided per day an increment from about 5% to about 200%;
   c. increasing a total of number of pulses provided per hour an increment from about 5% to about 200%;
   d. increasing a pulse amplitude of pulses provided an increment from about 5% to about 200%;
   e. increasing a pulse width of pulses provided an increment from about 5% to about 200%;
   f. increasing a number of pulses provided per cardiac cycle an increment from about 5% to about 200%.
Aspect 37: The system according to Aspect 36, wherein said increasing is performed by one or more of:
   a. automatically by an acute non-excitatory electrical heart failure device;
   b. manually by said patient.
Aspect 38: The system according to Aspect 29, wherein said controller is further configured to receive an indication of an improvement in said blood flow, and further configured for amending said effective amount of non-excitatory electrical heart failure therapy accordingly.
Aspect 39: The system according to Aspect 29, wherein said controller is further configured to receive an indication of an erection status in said patient.
Aspect 40: A system for cardiac stimulation, comprising an implantable controller, said controller configured to carry out the method of:
   a. receiving an indication that a patient has taken a specific pharmaceutical;
   b. providing an effective amount of non-excitatory electrical heart failure therapy.
Aspect 41: The system according to Aspect 40, wherein said system further comprises at least one sensor configured for detecting that said patient has taken said specific pharmaceutical; and wherein said indication is received from said at least one sensor.
Aspect 42: The system according to Aspect 41, wherein said at least one sensor is located within said system and implanted within a body of said patient.
Aspect 43: The system according to Aspect 41, wherein said at least one sensor is located outside a body of said patient and wherein said indication is transmitted to said implantable controller.
Aspect 44: The system according to Aspect 40, wherein said indication is provided to said implantable controller by an external source.
Aspect 45: The system according to Aspect 44, wherein said external source is one or more of a physician, said patient and an external device.
Aspect 46: The system according to Aspect 40, wherein said effective amount of non-excitatory electrical heart failure therapy comprises providing a pulse amplitude of 7.5V, a pulse width 5mS and two bipolar pulses per cardiac cycle.
Aspect 47: The system according to Aspect 40, wherein said providing comprises one or more of:
   a. providing said effective amount of non-excitatory electrical heart failure therapy for at least 5 hours of stimulation per day;
   b. increasing a total of number of pulses provided per day an increment from about 5% to about 200%;
   c. increasing a total of number of pulses provided per hour an increment from about 5% to about 200%;
   d. increasing a pulse amplitude of pulses provided an increment from about 5% to about 200%;
   e. increasing a pulse width of pulses provided an increment from about 5% to about 200%;
   f. increasing a number of pulses provided per cardiac cycle an increment from about 5% to about 200%.
Aspect 48: The system according to Aspect 47, wherein said increasing is performed by one or more of:
   a. automatically by an acute non-excitatory electrical heart failure device;
   b. manually by said patient.
Aspect 49: The system according to Aspect 40, wherein said controller is further configured to receive an indication of an improvement in a status in said patient, and further configured for amending said effective amount of non-excitatory electrical heart failure therapy accordingly.
Aspect 50: The system according to Aspect 40, wherein said controller is further configured to receive an indication of an erection status in said patient.
Aspect 51: A system for cardiac stimulation, comprising an implantable controller, said controller configured to carry out the method of:
   a. receiving an indication that a patient is suffering from an increase in stress levels;
   b. providing an effective amount of non-excitatory electrical heart failure therapy.
Aspect 52: The system according to Aspect 51, wherein said system further comprises at least one sensor configured for detecting said increase in stress levels; and wherein said indication is received from said at least one sensor.
Aspect 53: The system according to Aspect 52, wherein said at least one sensor is located within said system and implanted within a body of said patient.
Aspect 54: The system according to Aspect 52, wherein said at least one sensor is located outside a body of said patient and wherein said indication is transmitted to said implantable controller.
Aspect 55: The system according to Aspect 51, wherein said indication is provided to said implantable controller by an external source.
Aspect 56: The system according to Aspect 55, wherein said external source is one or more of a physician, said patient and an external device.
Aspect 57: The system according to Aspect 51, wherein said effective amount of non-excitatory electrical heart failure therapy comprises providing a pulse amplitude of 7.5V, a pulse width 5mS and two bipolar pulses per cardiac cycle.
Aspect 58: The system according to Aspect 51, wherein said providing comprises one or more of:
   a. providing said effective amount of non-excitatory electrical heart failure therapy for at least 5 hours of stimulation per day;
   b. increasing a total of number of pulses provided per day an increment from about 5% to about 200%;
   c. increasing a total of number of pulses provided per hour an increment from about 5% to about 200%;
   d. increasing a pulse amplitude of pulses provided an increment from about 5% to about 200%;
   e. increasing a pulse width of pulses provided an increment from about 5% to about 200%;
   f. increasing a number of pulses provided per cardiac cycle an increment from about 5% to about 200%.
Aspect 59: The system according to Aspect 58, wherein said increasing is performed by one or more of:
   a. automatically by an acute non-excitatory electrical heart failure device;
   b. manually by said patient.
Aspect 60: The system according to Aspect 51, wherein said controller is further configured to receive an indication of an improvement in said stress levels, and further configured for amending said effective amount of non-excitatory electrical heart failure therapy accordingly.
Aspect 61: The system according to Aspect 51, wherein said controller is further configured to receive an indication of an erection status in said patient.
Aspect 62: A system for cardiac stimulation, comprising an implantable controller, said controller configured to carry out the method of:
   a. receiving an indication that a patient is suffering of a change in temperature within a predetermined range;
   b. providing an effective amount of non-excitatory electrical heart failure therapy.
Aspect 63: The system according to Aspect 62, wherein said system further comprises at least one sensor configured for detecting said change in temperature; and wherein said indication is received from said at least one sensor.
Aspect 64: The system according to Aspect 63, wherein said at least one sensor is located within said system and implanted within a body of said patient.
Aspect 65: The system according to Aspect 63, wherein said at least one sensor is located outside a body of said patient and wherein said indication is transmitted to said implantable controller.
Aspect 66: The system according to Aspect 62, wherein said indication is provided to said implantable controller by an external source.
Aspect 67: The system according to Aspect 66, wherein said external source is one or more of a physician, said patient and an external device.
Aspect 68: The system according to Aspect 62, wherein said effective amount of non-excitatory electrical heart failure therapy comprises providing a pulse amplitude of 7.5V, a pulse width 5mS and two bipolar pulses per cardiac cycle.
Aspect 69: The system according to Aspect 62, wherein said providing comprises one or more of:
   a. providing said effective amount of non-excitatory electrical heart failure therapy for at least 5 hours of stimulation per day;
   b. increasing a total of number of pulses provided per day an increment from about 5% to about 200%;
   c. increasing a total of number of pulses provided per hour an increment from about 5% to about 200%;
   d. increasing a pulse amplitude of pulses provided an increment from about 5% to about 200%;
   e. increasing a pulse width of pulses provided an increment from about 5% to about 200%;
   f. increasing a number of pulses provided per cardiac cycle an increment from about 5% to about 200%.
Aspect 70: The system according to Aspect 69, wherein said increasing is performed by one or more of:
   a. automatically by an acute non-excitatory electrical heart failure device;
   b. manually by said patient.
Aspect 71: The system according to Aspect 62, wherein said controller is further configured to receive an indication of an improvement in said temperature, and further configured for amending said effective amount of non-excitatory electrical heart failure therapy accordingly.
Aspect 72: A system for cardiac stimulation, comprising an implantable controller, said controller configured to carry out the method of:
   a. receiving an indication that a patient is suffering from an acute response event to a previously provided amount of acute non-excitatory electrical heart failure therapy;
   b. amending at least one parameter of said previously provided amount of acute non-excitatory electrical heart failure therapy;
   c. providing an effective amount of amended non-excitatory electrical heart failure therapy.
Aspect 73: The system according to Aspect 72, wherein said system further comprises at least one sensor configured for detecting said acute response event; and wherein said indication is received from said at least one sensor.
Aspect 74: The system according to Aspect 73, wherein said at least one sensor is located within said system and implanted within a body of said patient.
Aspect 75: The system according to Aspect 73, wherein said at least one sensor is located outside a body of said patient and wherein said indication is transmitted to said implantable controller.
Aspect 76: The system according to Aspect 72, wherein said indication is provided to said implantable controller by an external source.
Aspect 77: The system according to Aspect 76, wherein said external source is one or more of a physician, said patient and an external device.
Aspect 78: The system according to Aspect 72, wherein said effective amount of amended non-excitatory electrical heart failure therapy comprises providing a pulse amplitude of 7.5V, a pulse width 5mS and two bipolar pulses per cardiac cycle.
Aspect 79: The system according to Aspect 72, wherein said providing comprises providing one or more of:
   a. providing said effective amount of non-excitatory electrical heart failure therapy for at least 5 hours of stimulation per day;
   b. increasing a total of number of pulses provided per day an increment from about 5% to about 200%;
   c. increasing a total of number of pulses provided per hour an increment from about 5% to about 200%;
   d. increasing a pulse amplitude of pulses provided an increment from about 5% to about 200%;
   e. increasing a pulse width of pulses provided an increment from about 5% to about 200%;
   f. increasing a number of pulses provided per cardiac cycle an increment from about 5% to about 200%.
Aspect 80: The system according to Aspect 79, wherein said increasing is performed by one or more of:
   a. automatically by an acute non-excitatory electrical heart failure device;
   b. manually by said patient.
Aspect 81: The system according to Aspect 72, wherein said controller is further configured to receive an indication of an improvement in said acute response event, and configured for amending again said effective amount of non-excitatory electrical heart failure therapy accordingly.

## Claims

1. A system for cardiac stimulation, comprising an implantable controller (812), said controller (812) configured to carry out a method **characterized by**:
- a. receiving an indication that a patient had suffered a myocardial infarction event and/or had heart surgery;
- b. providing an effective amount of non-excitatory electrical heart failure therapy.

2. The system according to claim 1, wherein said system further comprises at least one sensor (852) configured for detecting said myocardial infraction event; and wherein said indication is received from said at least one sensor (852).

3. The system according to claim 2, wherein said at least one sensor (852) is located within said system (800) and configured to be implanted within a body of said patient.

4. The system according to claim 2, wherein said at least one sensor is configured to be, during operation of the system for cardiac stimulation, located outside a body of said patient and wherein said system is configured to transmit said indication to said implantable controller (812).

5. The system according to claim 1, wherein said indication is provided to said implantable controller (812) by an external source.

6. The system according to claim 5, wherein said external source is one or more of a device operated by a physician, said patient and an external device.

7. The system according to claim 1, wherein said effective amount of non-excitatory electrical heart failure therapy comprises providing a pulse amplitude of 7.5V, a pulse width of 5ms and two bipolar pulses per cardiac cycle.

8. The system according to claim 1, wherein said providing an effective amount of non-excitatory electrical heart failure therapy comprises one or more of:
- a. providing said effective amount of non-excitatory electrical heart failure therapy for at least 5 hours of stimulation per day;
- b. increasing a total of number of pulses provided per day an increment from about 5% to about 200%;
- c. increasing a total of number of pulses provided per hour an increment from about 5% to about 200%;
- d. increasing a pulse amplitude of pulses provided an increment from about 5% to about 200%;
- e. increasing a pulse width of pulses provided an increment from about 5% to about 200%;
- f. increasing a number of pulses provided per cardiac cycle an increment from about 5% to about 200%.

9. The system according to claim 8, wherein said increasing is performed by one or more of:
- a. automatically by an acute non-excitatory electrical heart failure device;
- b. manually by said patient.

10. The system according to claim 1, wherein said controller (812) is further configured to receive a patient status improvement indication of an improvement in a status of said patient, and said controller (812) is further configured for amending said effective amount of non-excitatory electrical heart failure therapy in accordance with said patient status improvement indication.

11. The system according to any one of claims 1 to 10 further configured to operate as a cardioverter defibrillator (ICD).

12. The system according to claim 11 further comprising an ICD lead (822) and a cardiac contractility modulation lead (824).

13. The system according to claim 11 or 12 further comprising a defibrillation pulse generator (828) with a defibrillation capacitor (830).
